# EUROPÄISCHE PATENTANMELDUNG

(11) **EP 2 014 273 A1**
(43) Veröffentlichungstag der Anmeldung: **14.01.2009**
(21) Anmeldenummer: 07110259.4
(22) Anmeldetag: 14.06.2007
(51) Int. Cl.: A61K 8/04, A61K 8/33, A61Q 13/00, A61Q 15/00

(54) **Verwendung von Menthylmethylether und weiterer Ether zum Erzeugen eines Sauberkeits- und/oder Reinlichkeitsgefühls**

(71) Anmelder: Symrise GmbH & Co. KG, 37603 Holzminden (DE)
(72) Erfinder: Kurzenne, Pierre, 92270, Bois Colombes (FR); Collas, Violaine, 75018, Paris (FR); Favre-Bulle, Beatrice, 92200, Neuilly s/Seine (FR); Machinek, Arnold, 37603, Holzminden (DE); Surburg, Horst, 37603, Holzminden (DE)
(74) Vertreter: Stilkenböhmer, Uwe Michael

(57) **Zusammenfassung**

Die vorliegend Erfindung betrifft die Verwendung
(i) eines Ethers der Formel (1) oder
(ii) einer Mischung von zwei oder mehr unterschiedlichen Ethern der Formel (I) 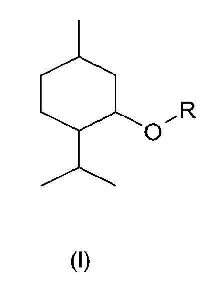 wobei R jeweils ein C₁ - C₄ - Alkyl darstellt,
zum Erzeugen eines Sauberkeits- und/oder Reinlichkeitsgefühls.

## Beschreibung

Die Erfindung betrifft die Verwendung bestimmter Ether des Menthols und seiner Isomere zum Erzeugen eines Sauberkeits- und/oder Reinlichkeitsgefühls. In diesem Zusammenhang betrifft die Erfindung insbesondere entsprechende Verwendungen, bei denen das Sauberkeits- und/oder Reinlichkeitsgefühl erzeugt wird durch die Wechselwirkung eines Ethers des Menthols oder dessen Isomeren mit einem Körpergeruch, wobei die dem Körpergeruch eigene olfaktorische Wirkung abgeschwächt wird und/oder mit einem Riechstoff, wobei die dem Riechstoff eigene olfaktorische Wirkung, insbesondere in Bezug auf die Fähigkeit, Körpergeruch zu überdecken, verstärkt wird. Das Sauberkeits-und/oder Reinlichkeitsgefühl kann dabei sowohl durch den Verwender selbst als auch durch andere Personen in dessen Umgebung wahrgenommen werden. Die Erfindung betrifft ferner die Verwendung der entsprechenden Ether zur Herstellung eines therapeutischen Mittels zum Einsatz gegen Körpergeruch und/oder dessen Folgen.

Darüber hinaus betrifft die Erfindung bestimmte Gemische, Parfümöle, kosmetische Zubereitungen und therapeutische Mittel, umfassend entsprechende Ether des Menthols und von dessen Derivaten und die Verwendung dieser Stoffgemische zum Erzeugen eines Sauberkeits- und/oder Reinlichkeitsgefühls. Außerdem betrifft die Erfindung entsprechende Verfahren.

Im Bereich der Kosmetik ist es für viele Anwendungen erwünscht, dass Kosmetika (kosmetische Zubereitungen) dem Anwender möglichst schnell ein Gefühl von Sauberkeit und Reinlichkeit vermitteln. Ohne einen entsprechenden Effekt bei beispielsweise Seifen, Haarwaschmitteln oder Deodorantien und Antiaspirantien würde eine Vielzahl von Verbrauchern auf konkurrierende Produkte zurückgreifen, die genau diesen Effekt vermitteln.

Das Gefühl von Sauberkeit und Reinlichkeit wird dabei im Regelfall durch Reize im Nasenraum ausgelöst. Ein entsprechendes Gefühl tritt insbesondere dann auf, wenn angenehme Gerüche überhaupt oder verstärkt wahrgenommen werden bzw. unangenehme Gerüche, insbesondere solche, die mit (körperlicher) Unsauberkeit verbunden sind, wie z. B. Schweißgeruch, nicht oder nicht mehr wahrgenommen werden.

Deodorantien und Antiperspirantien (Antiaspirantien) sind kosmetische Präparate, die zur Verhinderung und Beseitigung von unangenehmem Körpergeruch, besonders von Achselschweißgeruch, eingesetzt werden. Sie vermitteln ihrem Verwender das Gefühl und die Ausstrahlung von Sauberkeit und Reinlichkeit.

Die Wirkweise von Deodorantien und Antiperspirantien kann auf den unterschiedlichsten Mechanismen beruhen. So werden unterschiedliche Wirkstoffe eingesetzt wie z.B.
- deodorierend wirkende Substanzen (Deodorantien):
   antimikrobiell wirksame Substanzen, enzyminhibierende Substanzen, geruchsabsorbierende Substanzen, geruchsneutralisierende Substanzen und geruchsüberdeckende Substanzen; und
- schweißhemmende Stoffe (Antiperspirantien, Antitranspirantien).

Die Wirkung dieser Stoffe ist überwiegend darauf ausgelegt, über einen möglichst langen Zeitraum die Bildung von unangenehnem Körpergeruch bzw. dessen Wahrnehmung zu unterbinden.

Besonders bei bereits bestehendem Schweißgeruch ist es wünschenswert, wenn insbesondere die verwendeten Deodorantien beim Verwender zu einem möglichst unmittelbar einsetzenden und möglichst schnell wahrnehmbaren Sauber- und Reinlichkeitsgefühl führen. Ein solch schneller Effekt kann nur durch geruchsüberdeckende Substanzen, d.h. durch Parfüms mit einer entsprechend starken geruchlichen Wirkung erzielt werden. Wegen ihrer Stärke können diese Stoffe jedoch jeweils oft nur in jeweils ganz bestimmten Geruchstypen verwendet werden, die nicht immer von allen Verbrauchern mit einem Sauber- und Reinlichkeitsgefühl verbunden werden.

Zudem enthalten die meisten kosmetischen Zubereitungen mit Deodorant-, bzw. Antiperspirant-Wirkung viele schwer- bzw. nichtflüchtige Stoffe, die die Entfaltung der geruchsüberdeckenden Substanzen hemmen, indem ihre Freisetzung durch die fixierende Wirkung der höhermolekularen Matrix verzögert oder verhindert wird, wodurch ein unmittelbar einsetzendes Sauber- und Reinlichkeitsgefühl beeinträchtigt wird.

Aufgabe der vorliegenden Erfindung war es daher, Stoffe anzugeben, die insbesondere dem Anwender, aber auch Dritten ein über die Nase wahrnehmbares Sauberkeits- und Reinlichkeitsgefühl vermitteln können. Diese Stoffe sollten selbst über einen geringen Eigengeruch verfügen, so dass sie mit möglichst vielen Geruchstypen kombiniert werden können. Dabei sollte sich das Sauberkeits- und/oder Reinlichkeitsgefühl bevorzugt unabhängig vom verwendeten Geruchstyp einstellen und bevorzugt die Wirkung der eingesetzten Stoffe nicht durch andere, typischerweise in kosmetischen Zubereitungen enthaltene Stoffe beeinträchtigt werden. Bevorzugt sollte das Sauberkeits-und/oder Reinlichkeitsgefühl schnell wahrnehmbar sein. Insbesondere sollte das Sauberkeits- und Reinlichkeitsgefühl im Zusammenhang mit Deodorantien bzw. Antiperspirantien erzeugbar sein.

Überraschenderweise lassen sich diese erwünschten Effekte durch die Verwendung bestimmter Ether erzielen:
Dementsprechend wird erfindungsgemäß die vorgenannte Aufgabe gelöst durch Verwendung
   (i) eines Ethers der Formel (I) oder
   (ii) einer Mischung von zwei oder mehr unterschiedlichen Ethern der Formel (I) 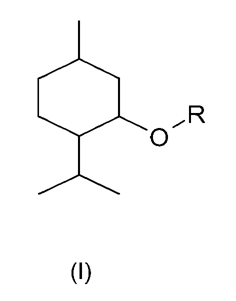
wobei R jeweils ein C₁ - C₄ - Alkyl darstellt, insbesondere -methyl, -ethyl, -n-propyl, -isopropyl, -n-butyl, -isobutyl, -2-butyl und -tert.-butyl.
zum Erzeugen eines Sauberkeits- und/oder Reinlichkeitsgefühls.

Im Rahmen dieses Textes ist unter einem Sauberkeits- und/oder Reinlichkeitsgefühl eine durch den Menschen über den Nasenraum, insbesondere über den Geruchssinn, wahrnehmbare Empfindung zu verstehen, die mit Sauberkeit/Reinlichkeit assoziiert ist. Dabei umfasst der Begriff Sauberkeits- und/oder Reinlichkeitsgefühl häufig die Verstärkung der Wahrnehmung von als positiv empfundenen Riechstoffen. Bei diesen Riechstoffen handelt es sich insbesondere um die weiter unten im Text explizit erwähnten Riechstoffe.

Im Rahmen dieses Textes beinhaltet ein Sauberkeits- und/oder Reinlichkeitsgefühl alternativ oder zusätzlich zu dem Vorgesagten auch die unterdrückte oder zumindest abgeschwächte Wahrnehmbarkeit negativ empfundener Gerüche insbesondere von Körpergerüchen. Ein Sauberkeits-und/oder Reinlichkeitsgefühl kann dementsprechend im Rahmen dieses Textes insbesondere durch das Abschwächen der Wahrnehmbarkeit von Körpergeruch bis hin zu nicht mehr wahrnehmbarer Stärke als auch durch das Verstärken von (bevorzugt angenehmen/sauber empfundenen) Gerüchen insbesondere in Bezug auf ihre Fähigkeit, negative Gerüche zu überdecken, erfolgen.

Die Verbindungen der Formel (I) haben sich in unseren Untersuchungen als Substanzen herausgestellt, die in Kombination mit den verschiedensten Geruchstypen ein Gefühl von Sauberkeit und/oder Reinlichkeit bei dem Anwender aber auch Dritten erzeugen können. Sie zeigten eine geruchsneutralisierende und/oder geruchsüberdeckende Wirksamkeit, insbesondere gegen Körpergeruch, ganz besonders gegen menschlichen Schweißgeruch, speziell gegen Achselschweißgeruch und/oder waren in der Lage, die weiter unten erwähnten, explizit benannten Riechstoffe hinsichtlich ihrer Wahrnehmbarkeit zu verstärken. Sofern im Rahmen des vorliegenden Textes nachfolgend Menthylether erwähnt sind, umfasst dieser Begriff die Ether des Menthols, des Neomenthols, des Isomenthols und/oder des Neoisomenthols. Die Ether der Formel (I) können als Racemate oder in enantiomerenreiner Form oder in einer entsprechenden Kombination vorliegen, wobei die entsprechenden Ether des Menthols [= (1α,2β,5α)-Menthol] bevorzugt sind.

Entsprechend sind erfindungsgemäß bevorzugte Menthylether der Formel (I) für die erfindungsgemäße Verwendung Ether des eigentlichen d-Menthols, des eigentlichen I-Menthols und Gemische der Ether des racemischen eigentlichen Menthols.

Dem Fachmann ist selbstverständlich klar, dass es sich bei einem Gemisch des Ethers des racemischen Menthols um ein Gemisch von zwei voneinander unterscheidbaren Ethern handelt. Sofern dieses "Gemisch" in einer Mischung von zwei oder mehr unterschiedlichen Ethern der Formel (I) vorliegt, die sich zueinander nicht wie Enantiomeren verhalten, wird aus Gründen der Lesbarkeit der Ether des racemischen Menthols so benannt, als handele es sich dabei um einen enantiomerenreinen Reinstoff (in diesem Fall wird gegebenenfalls das "Gemisch" oder Ether des racemischen Menthols als "ein Ether" bezeichnet).

Bevorzugt ist eine erfindungsgemäße Verwendung, wobei der, zumindest ein oder jeder Ether der Formel (I) jeweils ausgewählt ist aus der Gruppe bestehend aus I-Menthylmethylether, d-Menthylmethylether, dl-Menthylmethylether, I-Menthylethylether, d-Menthylethylether, dl-Menthylethylether, Menthyl-n-propylether, Menthylisopropylether und Menthylisobutylether.

Besonders bevorzugt ist die erfindungsgemäße Verwendung, wobei der, zumindest ein oder jeder Ether der Formel (I) jeweils ein Ether der Formel (II) ist, 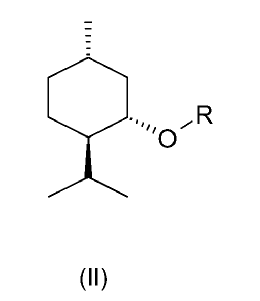 wobei R jeweils ein C₁ - C₄ - Alkyl darstellt, insbesondere -methyl, -ethyl, -n-propyl, -isopropyl, -n-butyl, -isobutyl, -2-butyl und -tert.-butyl.

Weiter bevorzugt ist eine erfindungsgemäße Verwendung, wobei bei dem, zumindest einem oder jedem der Ether der Formel (I) R jeweils Ethyl und/oder Methyl darstellt.

Am meisten bevorzugt ist eine erfindungsgemäße Verwendung, wobei der Ether der Formel (II) der I-Menthylmethylether ist, und zwar der Ether des eigentlichen I-Menthols, da diese Verbindung die ausgeprägteste geruchsneutralisierende und geruchsüberdeckende Wirkung zeigt und am stärksten und schnellsten ein Sauber- und Reinlichkeitsgefühl zu vermitteln vermag.

Bevorzugt ist eine erfindungsgemäße Verwendung, wobei das Sauberkeits-und/oder Reinlichkeitsgefühl erzeugt wird durch Wechselwirkung des, eines oder aller Ether der Formel (I)
(i) mit einem Körpergeruch, wobei die dem Körpergeruch eigene olfaktorische Wirkung abgeschwächt wird und/oder
(ii) mit einem Riechstoff, wobei die dem Riechstoff eigene olfaktorischen Wirkung, insbesondere in Bezug auf die Fähigkeit, Körpergeruch zu überdecken, verstärkt wird.

Im Falle, dass bei der letztgenannten erfindungsgemäßen Verwendung die Variante i) zutrifft, ist der Körpergeruch bevorzugt menschlicher Schweißgeruch, insbesondere Achselschweißgeruch.

Unter _{"}olfaktorischer Wirkung" sind in diesem Zusammenhang die über den Nasenraum, insbesondere den Geruchssinn wahrnehmbaren Sinneseindrücke zu verstehen.

Ether der Formel (I) und explizit I-Menthylmethylether sind aus WO 02/41861 bekannt. Diese Ether werden dort als Rhinologica [als Ersatz für Ersatz für 1,8-Cineol (Eucalyptol)] beschrieben, welche ein frisches befreiendes Gefühl im Mund, Rachenraum und den Atemwegen zu erzeugen vermögen. Zudem weisen sie frische, etherische, minzige, kühlende, süßliche und fruchtige Geschmacksnoten auf und sind daher auch hervorragend als Aromastoffe geeignet.

In US 6,231,900 werden bestimmte Ether als physiologische Kühlwirkstoffe beschrieben, welche ein Bestandteil von Süßwaren sein können, wie beispielsweise Pastillen oder Bonbons, die dort zur Linderung von Husten und Erkältungserscheinungen eingesetzt werden.

Keine der genannten Veröffentlichungen gibt Hinweise darauf, dass die erfindungsgemäß zu verwendenden Ether in der Lage sind, ein Sauberkeits-und/oder Reinlichkeitsgefühl zu vermitteln. Insbesondere geben die genannten Veröffentlichungen keinen Hinweis, dass Wechselwirkungen zwischen den erfindungsgemäß zu verwendenden Ethern und Riechstoffen und/oder Körpergerüchen bestehen könnten, die zu einer veränderten olfaktorischen Wahrnehmung der Riechstoffe bzw. des Körpergeruches führen. Dass die genannten Veröffentlichungen keine Hinweise zur Geruchswahrnehmung liefern können ist nachvollziehbar, da sie das technische Gebiet der Mundhygiene betreffen, das sich erheblich von dem hier einschlägigen technischen Fachgebiet der Parfümierung unterscheidet.

Bevorzugt eine erfindungsgemäße Verwendung wobei das Sauberkeits-und/oder Reinlichkeitsgefühl unmittelbar, d.h. innerhalb weniger Sekunden (regelmäßig innerhalb von weniger als 10 Sekunden) nach Applikation auf einen Menschen durch diesen und/oder einen Dritten wahrnehmbar ist.

Überraschenderweise hat sich gezeigt, dass die Empfindung des Sauberkeits-und/oder Reinlichkeitsgefühls bei entsprechender Applikation, insbesondere bei Applikation über Sprühen innerhalb kürzester Zeit einstellt. Das ist auch der Fall, wenn üblicherweise in Deodorantien verwendete Zusatzstoffe vorhanden sind. Diese Eigenschaft der erfindungsgemäß zu verwendenden Ether ist überraschend und macht sie besonders wertvoll, da ein schnelles Eintreten des beschriebenen Effektes in vielen Anwendungen wie z. B. Deos oder Seifen ausdrücklich erwünschst ist (vgl. dazu auch weiter oben).

Teil der Erfindung ist auch die Verwendung (i) eines Ethers der Formel (I), wie in einer der weiter oben erwähnten erfindungsgemäßen Verwendungen definiert, oder (ii) einer Mischung von zwei oder mehr unterschiedlichen Ethern der Formel (I), wie weiter oben in einer der erfindungsgemäßen Verwendungen definiert, zur Herstellung eines therapeutischen Mittels zum Einsatz gegen Körpergeruch und/oder dessen Folgen.

Körpergeruch, insbesondere Körpergeruch bei krankhaftem Schwitzen (Hyperhydrose), kann für das betroffene Individuum erhebliche, insbesondere auch psychologische Folgen haben. Daher eignet sich die erfindungsgemäße Verwendung auch zum Einsatz im medizinischen Bereich, insbesondere zur Prävention, Abmilderung oder Therapierung psychologischer Folgen von Körpergeruch, insbesondere von krankhaftem Körpergeruch.

Zur Erzielung eines bevorzugt unmittelbar wahrnehmbaren Sauberkeits- und Reinlichkeitsgefühls können die erfindungsgemäß einzusetzenden Ether der Formel (I) den kosmetischen Zubereitungen mit einer Deodorant-, bzw. Antiperspirant-Wirkung in reiner Form oder aber kombiniert mit weiteren Duftstoffen zugesetzt werden.

Dem Vorgesagten entsprechend ist Bestandteil der Erfindung ein Gemisch, umfassend
i) einen Ether der Formel (I), wie in einer der erfindungsgemäßen Verwendungen definiert, oder
ii) eine Mischung von zwei oder mehr unterschiedlichen Ethern der Formel (I), jeweils wie in einer der erfindungsgemäßen Verwendungen definiert, und
iii) wenigstens einen Riechstoff, wobei der Anteil an dem oder den Ethern der Formel (I) so eingestellt ist, dass die dem Riechstoff eigene olfaktorische Wirkung, insbesondere in Bezug auf die Fähigkeit, Körpergeruch zu überdecken, verstärkt ist.

### Bevorzugte Riechstoffe sind:

Extrakte aus natürlichen Rohstoffen wie Etherische Öle, Concretes, Absolues, Resine, Resinoide, Balsame, Tinkturen wie z. B.

Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl ; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl ; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl (engl.: pine oil); Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl (engl.: turpentine oil); Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen;

Einzel-Riechstoffe aus der Gruppe der Kohlenwasserstoffe, wie z. B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z. B. Hexanol; Octanol; 3-Octanol; 2,6-Dimethylheptanol; 2-Methyl-2-heptanol; 2-Methyl-2-octanol; (E)-2-Hexenol; (E)-und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z. B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
der aliphatischen Ketone und deren Oxime wie z. B. 2-Heptanon; 2-Octanon; 3-Octanon; 2-Nonanon; 5-Methyl-3-heptanon ; 5-Methyl-3-heptanonoxim; 2,4,4,7-Tetramethyl-6-octen-3-on; 6-Methyl-5-hepten-2-on;
der aliphatischen schwefelhaltigen Verbindungen wie z. B. 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z. B. 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
der Ester von aliphatischen Carbonsäuren wie z. B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat, ; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;4-Methyl-2-pentyl-crotonat;
der acyclischen Terpenalkohole wie z. B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone wie z. B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z. B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
der cyclischen Terpenaldehyde und -ketone wie z. B. Menthon; Isomenthon ; 8-Mercaptomenthan-3-on ; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on;2-Methyl-4-(2,6,6-trimethyl-1-cyclohexen-1-yl)-2-butenal; Nootkaton ; Dihydronootkaton ; 4,6,8-Megastigmatrien-3-on; alpha-Sinensal ; beta-Sinensal ; acetyliertes Cedernholzöl (Methylcedrylketon);
der cyclischen Alkohole wie z. B. 4-tert.-Butylcyclohexanol ; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z. B. alpha,3,3-Trimethylcyclohexylmethanol;1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z. B. Cineol; Cedrylmethylether; Cyclododecyl methylether;1,1-Dimethoxycyclododecan; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1 -methylpropyl)-1,3-dioxan;
der cyclischen und makrocyclischen Ketone wie z. B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
der cycloaliphatischen Aldehyde wie z. B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z. B. 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat; 2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Alkohole wie z. B.1-Cyclohexylethylcrotonat;
der Ester cycloaliphatischer Carbonsäuren wie z. B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole wie z. B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z. B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z. B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z. B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1 H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z. B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z. B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin;2-(3-Phenylpropyl)pyridin; Indol; Skatol; 2-Methoxy-3-isopropylpyrazin; 2-Isobutyl-3-methoxypyrazin;
der Phenole, Phenylether und Phenylester wie z. B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z. B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z. B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Aufgrund ihrer vergleichsweise neutralen Geruchsprofile können der oder die erfindungsgemäß zu verwendenden Ether der Formel (I) hervorragend und auf kompositorisch einfache Weise mit den oben genannten Riechstoffen der verschiedensten Duftrichtigungen zu erfindungsgemäßen Duftstoffkompositionen (wie beispielsweise Akkorden oder Parfümölen) kombiniert werden, ohne dass es dadurch zu einer problematischen Veränderung der sensorischen Eigenschaften der genannten Riechstoffe kommt. Solche Duftstoffkompositionen umfassen vorzugsweise mit zwei, drei, vier, fünf, sechs, sieben, acht, neun, zehn oder mehr der genannten Riechstoffe.

Die im Folgenden erwähnten Handelsnamen beziehen sich auf folgende Quellen:
¹ Handelsname Symrise GmbH, Deutschland;
² Handelsname Givaudan AG, Schweiz;
³ Handelsname International Flavors & Fragrances Inc., USA;
⁵ Handelsname Danisco Seillans S.A., Frankreich;
⁹ Handelsname Firmenich S.A., Schweiz;
¹⁰ Handelsname PFW Aroma Chemicals B.V., Niederlande.

Ferner haben unsere Untersuchungen gezeigt, dass erfindungsgemäße Gemische (erfindungsgemäße Akkorde) von einem oder mehreren erfindungsgemäß zu verwendenden Ethern der Formel (I) mit einem oder mehreren Riechstoffen der folgenden Gruppe (A) zu einer verbesserten Erzeugung eines bevorzugt schnell einsetzenden Sauberkeits- und Reinlichkeitsgefühls führen:
Gruppe (A):
   alpha-Hexylzimtaldehyd, 2-Phenoxyethylisobutyrat (Phenirat¹), Dihydromyrcenol (2,6-Dimethyl-7-octen-2-ol), Methyldihydrojasmonat; vorzugsweise mit einem Gehalt an cis-Isomerem > 60 Gew.-% (Hedione⁹, Hedione HC⁹), 4,6,6,7,8,8-Hexamethyl-1,3,4,6,7,8-hexahydrocyclopenta[g]benzopyran (Galaxolid³), Tetrahydrolinalool (3,7-Dimethyloctan-3-ol), Ethyllinalool, Benzylsalicylat, 2-Methyl-3-(4-tert-butyl-phenyl)propanal (Lilial²), Zimtalkohol, 4,7-Methano-3a,4,5,6,7,7a-hexahydro-5-indenylacetat und/oder 4,7-Methano-3a,4,5,6,7,7a-hexahydro-6-indenylacetat (Herbaflorat¹), Citronellol, Vanillin, Linalylacetat, Styrolylacetat (1-Phenylethylacetat), Octahydro-2,3,8,8-tetramethyl-2-acetonaphthon und/oder 2-Acetyl-1,2,3,4,6,7,8-octahydro-2,3,8,8-tetramethylnaphtalin (Iso E Super³), Hexylsalicylat, 4-tert.-Butylcyclohexylacetat (Oryclon¹), 2-tert.-Butylcyclohexylacetat (Agrumex HC ¹), alpha-lonon (4-(2,2,6-Trimethyl-2-cyclohexen-1-yl)-3-buten-2-on), n-alpha-Methylionon, alpha-Isomethylionon, Coumarin, Terpinylacetat, 4-(4-Hydroxy-4-methylpentyl)-3-cyclohexencarboxaldehyd (Lyral³), alpha-Amylzimtaldehyd, Ethylenbrassylat, (E)-und/oder (Z)-3-Methylcyclopentadec-5-enon (Muscenon⁹), 15-Pentadec-11-enolid und/oder 15-Pentadec-12-enolid (Globalide¹), 15-Cyclopentadecanolid (Macrolide¹), 1-(5,6,7,8-Tetrahydro-3,5,5,6,8,8-hexamethyl-2-naphthalenyl)ethanon (Tonalid¹⁰), 2-Isobutyl-4-methyltetrahydro-2H-pyran-4-ol (Florol⁹), 2-Ethyl-4- (2,2,3-trimethyl-3-cyclopenten-1-yl)-2-buten-1-ol (Sandolen¹).

In einer bevorzugten Ausführungsform werden daher die erfindungsgemäß zu verwendenden Ether der Formel (I) mit einem oder mehreren, vorzugsweise mit zwei, drei, vier, fünf, sechs oder mehr Riechstoffe der Gruppe (A) zu erfindungsgemäßen Gemischen (Akkorden) kombiniert, da solche Kombinationen ein besonders ausgeprägtes und gegebenenfalls unmittelbares Sauber- und Reinlichkeitsgefühl vermitteln können, wobei sie eine verbesserte geruchsneutralisierende / geruchsüberdeckende Wirkung zeigen und/oder die dem jeweiligen Riechstoff eigene olfaktorische Wirkung, insbesondere in Bezug auf die Fähigkeit, Körpergeruch zu überdecken, verstärken.

In den erfindungsgemäßen Gemischen (Akkorden) liegt dabei das Gewichtsverhältnis der Gesamtmenge an erfindungsgemäß zu verwendenden Ethern der Formel (I) zu der Gesamtmenge der Riechstoffe der Gruppe (A) vorzugsweise im Bereich von 15 : 1 bis 1 : 50, bevorzugt im Bereich von 8 : 1 bis 1 : 25, und besonders bevorzugt im Bereich von 4 : 1 bis 1 : 15.

In einer weiteren bevorzugten Ausführungsform werden die erfindungsgemäß zu verwendenden Ether der Formel (I) mit einem oder mehreren, vorzugsweise mit zwei, drei, vier, fünf oder mehr der im Folgenden genannten Riechstoffe der Gruppe (B) kombiniert, da solche Kombinationen ein besonders ausgeprägtes und unmittelbares Sauber- und Reinlichkeitsgefühl vermitteln können, wobei sie eine geruchsneutralisierende / geruchsüberdeckende Wirkung zeigen und/oder die dem jeweiligen Riechstoff eigene olfaktorische Wirkung, insbesondere in Bezug auf die Fähigkeit, Körpergeruch zu überdecken, verstärken:
Gruppe (B):
   cis-3-Hexenylacetat, trans-3-Hexenylacetat, trans-2,cis-6-Nonadienol, 2,4-dimethyl-3-cyclohexencarboxaldehyd (Vertocitral¹), 2,4,4,7-Tetramethyl-oct-6-en-3-on (Claritone¹), 5-Methyl-3-heptanonoxim (Stemone²), 2,6-Dimethyl-5-hepten-1-al (Melonal²), Borneol, 3-(3-Isopropylphenyl)butanal (Florhydral²), 2-Methyl-3-(3,4-methylendioxyphenyl)propanal (Helional³), 3-(4-Ethylphenyl)-2,2-dimethylpropanal (Florazon¹), 7-Methyl-2*H*-1,5-benzodioxepin-3(4*H*)-on (Calone 1951⁵), 2-sec-Butylcyclohexanon (Frescomenthe²), Orangenöl, Grapefruitöl, 3,3,5-Trimethylcyclohexylacetat (vorzugsweise mit einem Gehalt an cis-Isomeren größer oder gleich 70 Gew.-%), 2,5,5-Trimethyl-1,2,3,4,4a,5,6,7-octahydronaphthalin-2-ol (Ambrinol S¹), 2-Methyl-4-propyl-1,3-oxathiane (Oxane⁹).

In den erfindungsgemäßen Gemischen (Akkorden) liegt dabei das Gewichtsverhältnis der gewichtsbezogene Gesamtmenge an erfindungsgemäß zu verwendenden Ethern der Formel (I) zu der Gesamtmenge der Riechstoffe der Gruppe (B) vorzugsweise im Bereich von 50 : 1 bis 1 : 10, bevorzugt im Bereich von 25 : 1 bis 1 : 5, und besonders bevorzugt im Bereich von 10 : 1 bis 1 : 3.

Bei den erfindungsgemäßen Gemischen, umfassend einen oder mehrere erfindungsgemäß zu verwendende Ether der Formel (I) und einen oder mehrere Riechstoffe der Gruppe (A) und/oder (B) können regelmäßig Geruchsverstärkungen der Riechstoffe der Gruppe (A) und/oder (B) durch den oder die erfindungsgemäß zu verwendenden Ether der Formel (I) beobachtet werden (sogenannter Enhancer- oder Booster-Effekt), ohne dass es dabei zu einer nennenswerten Veränderung des Geruchsprofils des oder der Riechstoffe der Gruppe (A) und/oder (B) kommt.

Es wurde ferner gefunden, dass erfindungsgemäße Gemische umfassend einen oder mehrere erfindungsgemäß zu verwendende Ether der Formel (I) und einen oder mehrere Riechstoffe der Gruppe (A) und einen oder mehrere Riechstoffe der Gruppe (B) zu einem insgesamt verbesserten bevorzugt unmittelbaren Sauber- und Reinlichkeitsgefühl führen, bevorzugt mit geruchsneutralisierender /geruchsüberdeckender Wirkung, die besonders rasch eintritt, insbesondere wenn die Gemische in Form eines Sprays, speziell eines Aerosolsprays, vorliegen.

Es wurden ferner in eigenen Untersuchungen gefunden, dass bestimmte Kombinationen von erfindungsgemäß zu verwendenden Ethern der Formel (I), speziell von racemischem Menthylmethylether bzw. I-Menthylmethylether, mit ausgewählten Ausbringungshilfsmitteln und/oder ausgewählten Riechstoffen die Wirkung, insbesondere eine geruchsneutralisierende und geruchsüberdeckende Wirkung der erfindungsgemäß zu verwendenden Ether der Formel (I) weiter verbessert werden kann.

Aufgrund der oben genannten, insbesondere der geruchsverstärkenden, Eigenschaften und wegen ihrer ausgeprägten Diffusivität (Raumwirkung) eignen sich die Ether der Formel (I) bzw. erfindungsgemäße Gemische ferner zur Einarbeitung in Produkte zur Raumluftverbesserung (vorzugsweise in flüssiger, gelartiger oder auf einem festen Träger aufgebrachter Form sowie Pumpsprays oder Aerosolsprays), wie Raumsprays, (WC-)Lufterfrischer, Duftbäume usw..

Ether der Formel (I) bzw. erfindungsgemäße Gemische können dabei in manchen Fällen auch anderen unangenehmen Gerüchen entgegenwirken und/oder diese überdecken wie beispielsweise Fäkalien- und Uringerüche von Menschen oder (Haus)Tieren, Küchengerüche, wie sie z.B. bei der Zubereitung von Zwiebeln, Knoblauch, Kohl oder Fisch entstehen, Gerüche von Tabakrauch und von Haushaltsabfällen.

Bevorzugte Kombinationen mit Ausbringungshilfsmitteln der Gruppe (H), insbesondere für eine weiter verbesserte Wirksamkeit der erfindungsgemäß zu verwendenden Ether der Formel (I) aus Deo-Sprays heraus, speziell Aerosolsprays, umfassen eine oder mehrere Substanzen aus der Gruppe (H) bestehend aus Dipropylenglycol (DPG), Diethylphthalat (DEP), Triethylcitrat (TEC), Isopropylmyristat (IPM), und/oder Benzylbenzoat (BB). Solche Mischungen sind neu.

Vorzugsweise liegt das Massenverhältnis der Gesamtmenge der erfindungsgemäß zu verwendenden Ether der Formel (I) zu der Gesamtmenge der Ausbringungshilfsmittel der Gruppe (H) im Bereich von 3 : 1 bis 1 : 100, bevorzugt im Bereich von 1 : 1 bis 1 : 20, am meisten bevorzugt im Bereich von 1 : 2 bis 1 : 10.

Um den Eindruck des gewünschten unmittelbaren Sauber- und Reinlichkeitsgefühls sowie der geruchsneutralisierenden /geruchsüberdeckenden Wirkung der erfindungsgemäß zu verwendenden Ether der Formel (I) weiter zu steigern, ist neben einem oder mehreren Riechstoffen der Gruppe (A) und/oder der Gruppe (B) die zusätzliche Anwesenheit mindestens einer Komponente der folgenden Gruppe (M) bevorzugt:

Eucalyptus-globulus-Öl, Pfefferminzöl, Krauseminzöl, Menthylacetat, Salbeiöl, I-Carvon, Menthol (vorzugsweise I-Menthol oder racemisches Menthol, besonders bevorzugt I-Menthol), Anethol, alpha-Terpineol, Geraniol, Linalool, 2-Phenylethylalkohol.

In den erfindungsgemäßen Gemischen (Akkorden) liegt dabei das Gewichtsverhältnis der Gesamtmenge an erfindungsgemäß zu verwendenden Ethern der Formel (I) zu der Gesamtmenge der Riechstoffe der Gruppe (M) vorzugsweise im Bereich von 10 : 1 bis 1 : 10, bevorzugt im Bereich von 5 : 1 bis 1 : 5, und besonders bevorzugt im Bereich von 2 : 1 bis 1 : 2. Das Gewichtsverhältnis der Gesamtmenge an erfindungsgemäß zu verwendenden Ethern der Formel (I) zu der Gesamtmenge an Menthol, insbesondere zu I-Menthol, liegt dabei vorzugsweise im Bereich von 4 : 1 bis 1 : 4, bevorzugt im Bereich von 2 : 1 bis 1 : 2.

Einzelne zur Verwendung im Rahmen der vorliegenden Erfindung (in erfindungsgemäßen Gemischen) bevorzugte Kühlwirkstoffe sind nachfolgend aufgelistet. Die aufgelisteten Kühlwirkstoffe können auch in Kombination miteinander eingesetzt werden: Menthonglycerinacetal (Handelsname: Frescolat^{®}MGA), Menthyllactat (Handelsname: Frescolat^{®}ML, vorzugsweise handelt es sich bei Menthyllactat um I-Menthyllactat, insbesondere I-Menthyl-I-lactat), substituierte Menthyl-3-carbonsäureamide (z.B. Menthyl-3-carbonsäureN-ethylamid), 2-Isopropyl-N-2,3-trimethylbutanamid, substituierte Cyclohexancarbonsäureamide, 3-Menthoxypropan-1,2-diol, 2-Hydroxyethylmenthylcarbonat, 2-Hydroxypropylmenthylcarbonat, N-Acetylglycinmenthylester, Isopulegol, Menthylhydroxycarbonsäureester (z.B. Menthyl-3-hydroxybutyrat), Monomenthylsuccinat, 2-Mercaptocyclodecanon, Menthyl-2-pyrrolidin-5-oncarboxylat, 2,3-Dihydroxy-p-menthan, 3,3,5-trimethylcyclohexanonglycerinketal, 3-Menthyl-3,6-di- und -trioxaalkanoate, 3-Menthyl methoxyacetat, Icilin.

Bevorzugte Kühlwirkstoffe sind: Menthonglycerinacetal (Handelsname: Frescolat^{®}MGA), Menthyllactat (vorzugsweise I-Menthyllactat, insbesondere I-Menthyl-I-lactat, Handelsname: Frescolat^{®}ML), substituierte Menthyl-3-carbonsäureamide (z.B. Menthyl-3-carbonsäure-N-ethylamid), 2-Isopropyl-N-2,3-trimethylbutanamid, substituierte Cyclohexancarbonsäureamide, 3-Menthoxypropan-1,2-diol, 2-Hydroxyethylmenthylcarbonat, 2-Hydroxypropylmenthylcarbonat, Isopulegol.

Besonders bevorzugte Kühlwirkstoffe sind: Menthonglycerinacetal (Handelsname: Frescolat^{®}MGA), Menthyllactat (vorzugsweise I-Menthyllactat, insbesondere I-Menthyl-I-lactat, Handelsname: Frescolat^{®}ML), 3-Menthoxypropan-1,2-diol, 2-Hydroxyethylmenthylcarbonat, 2-Hydroxypropylmenthylcarbonat.

Ganz besonders bevorzugte Kühlwirkstoffe sind: Menthonglycerinacetal (Handelsname: Frescolat®MGA), Menthyllactat (vorzugsweise I-Menthyllactat, insbesondere I-Menthyl-I-lactat, Handelsname: Frescolat^{®}ML).

Teil der Erfindung ist auch ein Parfümöl zum Erzeugen eines bevorzugt schnell einsetzenden Sauberkeits- und Reinlichkeitsgefühls, umfassend ein erfindungsgemäßes Gemisch. Insbesondere sind in solchen Parfümölen die olfaktorischen Wirkungen eines, mehrer oder aller im Parfümöl enthaltenen Riechstoffe (insbesondere die weiter oben explizit erwähnten) verstärkt und/oder besitzt das Parfümöl eine geruchsneutralisierende/geruchsüberdeckende Wirkung gegenüber Körpergeruch, insbesondere Schweißgeruch.

Ein wesentlicher Vorteil der erfindungsgemäßen Parfümöle besteht darin, dass die erfindungsgemäß zu verwendenden Ether der Formel (I) aufgrund ihres geringen Eigengeruches mit diesem Eigengeruch nur wenig, bevorzugt überhaupt nicht zum Gesamtgeruchseindruck beitragen.

In einem erfindungsgemäßen Parfümöl liegt die Gesamtmenge an erfindungsgemäß zu verwendenden Ethern der Formel (I) im Bereich von 0,5 bis 30 Gew.-%, bevorzugt im Bereich von 1 bis 20 Gew.-% und besonders bevorzugt im Bereich von 2 bis 15 Gew.-%, bezogen auf die Gesamtmasse des Parfümöls.

Bestandteil der Erfindung sind auch kosmetische Zubereitungen, die ein erfindungsgemäßes Parfümöl oder ein erfindungsgemäßes Gemisch umfassen. Bevorzugte erfindungsgemäße kosmetische Zubereitungen umfassen ein erfindungsgemäßes Parfümöl in einer Menge im Bereich von 0,1 bis 5 Gew.-%, bevorzugt im Bereich von 0,2 bis 3 Gew.-%, und besonders bevorzugt im Bereich von 0,3 bis 2 Gew.-%, bezogen auf die Gesamtmasse der kosmetischen Zubereitung.

Wie auch die erfindungsgemäßen Parfümöle besitzen die erfindungsgemäßen kosmetischen Zubereitungen den Vorteil, dass aufgrund der weitgehenden Geruchsneutralität der erfindungsgemäß zu verwendenden Ether der Formel (I) die erfindungsgemäßen kosmetischen Zubereitungen nicht an bestimmte Geruchstypen gebunden sind und somit hinsichtlich ihrer tatsächlichen Zusammensetzung durch die erfindungsgemäß zu verwendenden Ether der Formel (I) nur wenig oder gar nicht eingeschränkt werden. Bevorzugt ist bei solchen kosmetischen Zubereitungen, dass ein wahrnehmbares Sauberkeits-und Reinlichkeitsgefühl schnell auftritt. Es kann aber auch wünschenswert sein, dass die erfindungsgemäß zu verwendenden Ether der Formel (I) ihre Wirkung verzögert oder z. B. erst bei Auftreten von Körperschweiß ausüben. Dies kann durch geeignete Maßnahmen bei der Einbindung der entsprechenden Ether in die erfindungsgemäßen Gemische, Parfümöle oder kosmetischen Zubereitungen erreicht werden (vgl. dazu auch weiter unten).

Bevorzugte erfindungsgemäße kosmetische Zubereitungen liegen in einer Applikationsform vor, ausgewählt aus der Gruppe bestehend aus Stiften, Cremes, Gelen, Lotionen, Schäumen, Roll-on-Präparaten (z. B. Roll-on-Gele, Roll-on-Emulsionen), Pudersprays, Areosol- oder Non-Areosol-Sprays (Pumpspray). Bevorzugt sind Sprays, dabei wiederum insbesondere bevorzugt Aerosolsprays, da (Aerosol-)Sprays, die die erfindungsgemäß zu verwendenden Ether der Formel (I) umfassen (insbesondere 1-Menthylmethylether) am stärksten und schnellsten ein Sauberkeits- und Reinlichkeitsgefühl vermitteln, wobei sie eine hervorragende neutralisierende und geruchsüberdeckende Wirkung gegen Schweißgeruch zeigen und/oder die olfaktorische Wirkung eines oder mehrerer oder aller in der kosmetischen Zubereitung vorhandenen Riechstoffe, insbesondere in Bezug auf die Fähigkeit, Körpergeruch zu überdecken, verstärken.

Dementsprechend sind besonders bevorzugte erfindungsgemäße kosmetische Zubereitungen Deodorantien und/oder Antiaspirantien.

Die mit dem oder den erfindungsgemäß zu verwendenden Ethern der Formel (I), bzw. mit erfindungsgemäßen Gemischen oder erfindungsgemäßen Parfümölen versetzten kosmetischen Deodorantien und Antiperspirantien können je nach ihrer Wirkweise einen oder mehrere der folgenden Wirkstoffe enthalten:
1.) Deodorantien
   1-1.) Antimikrobiell wirksame Substanzen, die die Entwicklung der für den Schweißgeruch verantwortlichen Mikroorganismen hemmen. Beispiele hiefür sind Triclosan^{®} (5-Chlor-2-(2,4-dichlorphenoxy)phenol), Triclocarban, Chlorhexidin, Chlorhexidinhydrochlorid, Chlorhexidindiacetat, Chlorhexidindigluconat, 2-Phenoxyethanol, Farnesol, Glycerinester und -ether wie Glycerinmonolaurat, Glycerinmonocaprinat, Hexoxyglycerin, Octoxyglycerin (= Ethylhexylglycerin, 3-(2-Ethylhexyloxy-1,2-Propandiol), z.B. Sensiva^{®} SC 50 von Schülke & Mayr), aliphatische 1,2-Diole wie z.B. 1,2-Decandiol (EP 1 269 983), araliphatische Alkohole wie beispielsweise 2-Methyl-5-phenylpentan-1-ol (Rosaphen¹) oder solche wie beschrieben in EP 799 174, dabei vorzugsweise 4-Methyl-4-phenyl-2-pentanol (Vetikol; WO 03/024907), 2-Benzylheptan-1-ol (Jasmol¹; 2-n-Pentyl-3-phenylpropan-1-ol), 2,2-Dimethyl-3-phenylpropanol (Muguetalkohol¹; vgl. US 4,091,090), antimikrobiell wirksame sekundäre Alkohol, wie beispielsweise beschrieben in WO 2005/004601, insbesondere 3-Methyl-6-phenyl-2-hexanol, 4-(2,4-Dimethylphenyl)-2-butanol, 6-(4-Isopropylphenyl)-3-methyl-2-hexanol , 4-(2,4,5-Trimethylphenyl)-2-butanol, 3,3-Dimethyl-4-phenyl-2-butanol, 3-Methyl-4-(2-methylphenyl)-2-butanol, 3-Methyl 4-(2,4,5-trimethylphenyl)- 2 -butanol, 3,3-Dimethyl-4-(2,5-dimethylphenyl)-2-butanol, 3-Methyl-4-(4-methylphenyl)-2-butanol, 4-(3,4-Dimethylphenyl)-3-methyl-2-butanol, 4-(3,4-Dimethylphenyl)-2-butanol, 4-(2,4,6-Trimethylphenyl)-2-butanol, 4-Bicyclo[2.2.1]hept-2-yl-2-butanol, 3-Methyl-4-(2,4,6-trimethylphenyl)-2-butanol, 4-(2,6,6-Trimethylbicyclo[3.1.1]hept-3-yl)-2-butanol, 5,5-Dimethyl-6-(3-methylphenyl)-hexan-2-ol, 4-Bicyclo[2.2.1]hept-2-yl-3-methyl-2-butanol, 3,3-Dimethyl-4-(2,4,6-trimethylphenyl)-2-butanol, 4-(4-t-Butylphenyl)-3-methyl-2-butanol, 4-(4-Isopropylphenyl)-3-methyl-2-butanol, 6-(3,4-Dimethylphenyl)-3-methyl-2-hexanol, 4-(2,4-Dimethylphenyl)-3-methyl-2-butanol, 6-(3,4-Dimethylphenyl)-2-hexanol, aliphatische Carbonsäuren wie 2-Hexyloctansäure, 2-Hexyldecansäure, 2-Butyloctansäure oder 2-Butyldecansäure.
   1-2.) Enzyminhibierende Substanzen, die die Wirkung von Enzymen, die an der Bildung des Schweißgeruches beteiligt sind, unterbinden. Beipsiel hiefür sind Citronensäureester und metall-chelatisierende Substanzen wie EDTA (Ethylendiamintetraessigsäure), EGTA [Ethylenbis(oxyethylennitrilo)-tetraessigsäure] und DTPA (Diethylentriaminpentaessigsäure, Pentetic acid).
   1-3.) Geruchsabsorbierende Substanzen, die die für den Schweißgeruch verantwortlichen Stoffe absorbieren. Beispiel hierfür ist z.B. Zinkrizinoleat.
   1-4.) Geruchsneutralisierende Substanzen, die auf Grund spezieller Eigenschaften in der Lage sind, die geruchliche Wahrnehmung herabzusetzen. Beispiele hierfür sind z.B. in WO 01/43784 genannt, bevorzugt ist dabei Isomenthylacetat.
   1-5.) Geruchsüberdeckende Substanzen, die wegen ihres starken Geruchs und ihres Geruchstyps in der Lage sind, den Schweißgeruch zu überdecken. Beispiele hierfür sind z.B. in der WO 2006/124230 genannt. In wasserfreien kosmetischen Zubereitungen können diese Substanzen in sprühgetrockneter oder eingekapselter Form, z. B. durch Cyclodextrine, verwendet werden.
2.) Antiperspirantien

Antiperspirantien hemmen die Schweißsekretion und entziehen damit den für den Körpergeruch verantwortlichen Bakterien den Nährboden. Als Antiperspirantien verwendet man im allgemeinen adstringierende Metallsalze, besonders anorganische und organische Metallsalze der Elemente Aluminium, Zink, Magnesium, Zinn und Zircon sowie deren Mischungen, wobei insbesonders Halogenide wie Aluminiumchlorid, basische Aluminiumhydroxychloride, Zirconyloxichloride und Zirconylhydroxychloride sowie deren Mischungen verwendet werden. Häufig werden diese Aluminium- und Zirconiumsalze und deren Mischungen auch in einer komplexierten Form verwendet, wobei als Komplexbildner Propylenglycol, Polyethylenglycol oder Glycin verwendet werden.

Folgende Antiperspirantien oder deren Mischungen sind dabei bevorzugt:
Aluminiumchlorohydrat, Aluminiumsesquichlorohydrat, Aluminiumchlorohydrex PG, Aluminiumdichlorohydrex PG, Aluminiumsesquichlorohydrex PG, Aluminiumchlorohydrex PEG, Aluminiumsesquichlorohydrex PEG, Aluminiumchlorid (vorzugsweise in Form einer bis zu 15 Gew.-%igen (wässrigen) Lösung), Aluminium Zirconium Chlorohydrat, Aluminium Zirconium Trichlorohydrat, Aluminium Zirconium Tetrachlorohydrat, Aluminium Zirconium Pentachlorohydrat, Aluminium Zirconium Octachlorohydrat, Aluminium Zirconium Trichlorohydrex Glycin (Gly), Aluminium Zirconium Pentachlorohydrex Gly, Aluminium Zirconium Tetrachlorohydrex Gly, Aluminium Zirconium Octachlorohydrex Gly, gepuffertes Aluminiumsulfat, basische Aluminiumchloride, Zirconiumhydroxychlorid, Zirconylchlorid, basische Aluminiumnitrate, basische Aluminiumchloride kombiniert mit Zirconyloxychlorid und/oder -hydroxychlorid, organische Komplexe von basischen Aluminiumchloriden und/oder Zirconylchlorid und/oder Zirconiumhydroxychlorid.

Bevorzugt sind dabei wiederum Aluminum oder Aluminum Zirconium Komplexe mit einem Metall/Anion Verhältnis im Bereich von 0,9 : 1 bis 2,1 : 1, wobei das Anion vorzugsweise gewählt ist aus der Gruppe, bestehend aus Cl⁻, Br⁻, I⁻ und/oder NO₃⁻, gegebenenfalls in Kombination mit Additiven wie Aminosäuren (dabei vorzugsweise Glycin) oder ein- oder mehrwertigen Alkoholen. Die mehrwertigen Alkohole sind vorzugsweise Di-, Tri- oder Polyole mit 3 bis 12 C-Atomen, dabei wiederum bevorzugt sind Glycerin, Propylenglycol, Diglycerin, Tripropylenglycol, Sorbitol, 1,2,4-Butantriol oder 1,2,6-Hexantriol und deren Mischungen, besonders bevorzugt sind Glycerin und Diglycerin. Vorteilhafte Monoalkohole sind Glycolether wie Monoalkylether oder alpha-Hydroxysäuren wie Milchsäure.

Im Rahmen der vorliegenden Erfindung können auch Mischungen von Antiperspirantien umfassend (i) Aluminium und/oder Zirconium und (ii) Zink und/oder Zinn eingesetzt werden, wie beispielsweise Al/Zr/Zn, Al/Zn, Al/Sn oder Al/Zr/Sn, wobei Mischungen umfassend ein oder mehrere Antiperspirantien der oben bevorzugt genannten Antiperspirantien basierend auf Aluminium und/oder Zirconium wiederum bevorzugt sind.

Gegebenenfalls können die erfindungsgemäßen kosmetischen Deodorantien und Antiperspirantien neben den oben beschriebenen speziellen Wirkstoffen weitere Stoffe enthalten:
Treibgase, Ethanol, Propylenglycol, Emulgatoren wie z.B. Aminomethylpropanol, hautpflegende/rückfettende Substanzen wie z.B. 2-Octyldodecanol, Isopropylmyristat, Isopropylpalmitat, Stearamid, Sorbit, Glycerin sowie modifizierte Polyethylen- und Polypropylenglycole, Vitamine und deren Derivate (z.B. Tocopherol (Vitamin E), Tocopherylacetat (Vitamin E - Acetat) und Ascorbinsäure (Vitamin C)), Panthenol, Allantoin, Pflanzenextrakte, beispielsweise Aloe Vera und Proteine, Perlglanzmittel, Elektrolytsalze wie KCI, NaCl, Gel-bildende Substanzen wie z.B. Hydroxyalkylcellulosen, Fettalkohole, Fettsäuren, Fettalkoholfettsäureester, Fettsäureglycerylester o.ä., flüssige Trägerstoffe und Lösungsmittel wie z.B. flüchtige und nichtflüchtige Siliconöle, feste Trägerstoffe wie z.B. Talkum, Kieselgele o.ä.; Antioxidantien und Konservierungsstoffe, UV-Lichtschutzfilter, Kühlwirkstoffe, weitere Parfümrohstoffe, um Geruchstypen zu modifizieren, die sensorische Akzeptanz zu erhöhen und/oder das hedonistische Empfinden zu verbessern.

Eine einen erfindungsgemäß zu verwendenden Ether der Formel (I) oder mehrere entsprechende Ether der Formel (I) enthaltende erfindungsgemäße Duftstoffkomposition (ein erfindungsgemäßes Gemisch / ein erfindungsgemäßes Parfümöl) kann den kosmetischen Zubereitungen mit Deodorant-, bzw Antiperspirantwirkung in reiner Form oder aber beispielsweise auch in geträgerter oder verkapselter Form zugesetzt werden.

Für manche Anwendungen ist es vorteilhaft, eine einen erfindungsgemäß zu verwendenden Ether der Formel (I) oder mehrere erfindungsgemäß zu verwendende Ether der Formel (I) enthaltende erfindungsgemäße Duftstoffkomposition an einem Trägerstoff adsorbiert einzusetzen, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer, Cellulosebasierende Stoffe, Zucker oder Kunststoffe wie PVC, Polyvinylacetate oder Polyurethane sein.

Für andere Anwendungen ist es vorteilhaft, eine einen erfindungsgemäß zu verwendenden Ether der Formel (I) oder mehrere erfindungsgemäß zu verwendende Ether der Formel (I) enthaltende Duftstoffkomposition mikroverkapselt, sprühgetrocknet, als Einschluss-Komplex oder als Extrusions-Produkt einzusetzen und in dieser Form dem zu parfümierenden (Vor-)Produkt hinzufügen.

Die Eigenschaften derart modifizierter Riechstoffmischungen (Parfümöle) werden in manchen Fällen durch sogenanntes "Coaten" mit geeigneten Materialien im Hinblick auf eine gezieltere Duftfreisetzung weiter optimiert, wozu vorzugsweise wachsartige Kunststoffe wie z. B. Polyvinylalkohol verwendet werden.

Die Mikroverkapselung der einen erfindungsgemäß zu verwendenden Ether der Formel (I), oder mehrere erfindungsgemäß zu verwendende Ether der Formel (I) enthaltenden Duftstoffkompositionen kann beispielsweise durch das sogenannte Koazervationsverfahren mit Hilfe von Kapselmaterialien z. B. aus polyurethanartigen Stoffen oder Weichgelatine, erfolgen. Die sprühgetrockneten erfindungsgemäßen Parfümöle können beispielsweise durch Sprühtrocknung einer das Parfümöl enthaltenden Emulsion, bzw. Dispersion hergestellt werden, wobei als Trägerstoffe modifizierte Stärken, Proteine, Dextrin und pflanzliche Gummen verwendet werden können. Einschluss-Komplexe können z. B. durch Eintragen von Dispersionen von dem Parfümöl und Cyclodextrinen oder Harnstoffderivaten in ein geeignetes Lösungsmittel, z. B. Wasser, hergestellt werden. Extrusions-Produkte können durch Verschmelzen der Parfümöle mit einem geeigneten wachsartigen Stoff und durch Extrusion mit nachfolgender Erstarrung, ggf. in einem geeigneten Lösungsmittel, z. B. Isopropanol, erfolgen.

Teil der Erfindung ist auch ein therapeutisches Mittel zur Behandlung von Körpergeruch und/oder dessen Folgen, umfassend ein erfindungsgemäßes Gemisch oder ein erfindungsgemäßes Parfümöl. Einsatzbereich eines solchen Therapeutikums im medizinischen Bereich (vgl. auch weiter oben).

Entsprechend dem Vorgesagten ist ebenfalls Teil der Erfindung die Verwendung eines erfindungsgemäßen Gemisches oder eines erfindungsgemäßen Parfümöls oder einer erfindungsgemäßen Zubereitung zum Erzeugen eines Sauberkeits-und/oder Reinlichkeitsgefühls.

Dabei wird das Sauberkeits- und/oder Reinlichkeitsgefühl bevorzugt erzeugt durch Wechselwirkung des, eines oder aller erfindungsgemäß zu verwendenden Ether der Formel (I),
(i) mit einem Körpergeruch, wobei die dem Körpergeruch eigene olfaktorische Wirkung abgeschwächt wird und/oder
(ii) mit einem Riechstoff, wobei die dem Riechstoff eigene olfaktorischen Wirkung, insbesondere in Bezug auf die Fähigkeit, Körpergeruch zu überdecken, verstärkt wird,
wobei der Körpergeruch bevorzugt menschlicher Schweißgeruch, insbesondere Achselschweißgeruch ist.

Erfindungsgemäß bevorzugt ist die Verwendung eines erfindungsgemäßen Gemisches, eines erfindungsgemäßen Parfümöls oder einer erfindungsgemäßen Zubereitung, wobei das Sauberkeits- und/oder Reinlichkeitsgefühl unmittelbar, d.h. innerhalb von weniger Sekunden (regelmäßig innerhalb von weniger als 10 Sekunden, bevorzugt 5 Sekunden), nach Applikation auf einen Menschen durch diesen und/oder einen Dritten wahrnehmbar ist.

Teil der Erfindung ist auch die Verwendung eines erfindungsgemäßen Gemisches, eines erfindungsgemäßen Parfümöls oder einer erfindungsgemäßen Zubereitung zur Herstellung eines therapeutischen Mittels zum Einsatz gegen Körpergeruch und/oder dessen Folgen.

Entsprechend dem Vorgesagten ist ebenfalls Teil der Erfindung ein Verfahren zum kosmetischen Erzeugen eines Sauberkeits- und/oder Reinlichkeitsgefühls, umfassend die Schritte:
a) Bereitstellen
   (i) eines erfindungsgemäß zu verwendenden Ethers der Formel (I) oder
   (ii) einer Mischung von zwei oder mehr unterschiedlichen erfindungsgemäß zu verwendenden Ethern der Formel (I) oder
   (iii) ein erfindungsgemäßes Gemisch oder
   (iv) ein erfindungsgemäßes Parfümöl oder
   (v) eine erfindungsgemäße Zubereitung und
b) Applizieren, des Ethers, der Mischung der Ether, des Gemisches, des Parfümöls oder der Zubereitung, so dass die olfaktorische Wirkung des Ethers, der Mischung der Ether, des Gemisches, des Parfümöls oder der Zubereitung so wahrnehmbar ist, dass ein Sauberkeits- und/oder Reinlichkeitsgefühls erzeugt wird.

Bevorzugt bei dem erfindungsgemäßen Verfahren ist, dass das Applizieren auf die Haut erfolgt.

In vielen Fällen ist ein erfindungsgemäßes Verfahren bevorzugt, bei dem das Applizieren in Anwesenheit von wahrnehmbaren Körpergeruch erfolgt. Ebenso ist bevorzugt bei erfindungsgemäßen Verfahren, dass das Applizieren im Bereich einer Quelle oder eine potentiellen Quelle vom Körpergeruch erfolgt. Als Quelle bzw. potentielle Quelle sind insbesondere die Achselhöhlen zu verstehen.

Bei einem besonders bevorzugten erfindungsgemäßen Verfahren erfolgt das Applizieren durch Sprühen.

Nachfolgend wird die Erfindung bzw. einzelne Aspekte davon durch die Ansprüche und die Beispiele weiter erläutert.

### Beispiele

Sofern nicht anders angegeben beziehen sich alle Angaben auf das Gewicht.

### Beispiel 1. Parfümöl zur Verwendung in Deodorantien und Antiperspirantien (nicht erfindungsgemäß)

Es werden vermischt:

| Komponente | Gew.-teile | Gruppe |
|---|---|---|
| 10-Undecenal 10% in DPG | 2 | H |
| Vertocitral¹ | 0,5 | B |
| cis-3-Hexenylacetat 10% in DPG | 7,5 | B, H |
| Allylamylglycolat³ | 2 | |
| Melonal² | 0,5 | B |
| Bergamotteöl | 80 | |
| Dihydromyrcenol | 90 | A |
| Terpinylacetat | 40 | A |
| Litsea cubeba Öl | 2 | |
| Citronenöl | 50 | |
| Orangenöl | 20 | A |
| Grapefruitöl | 10 | A |
| Lavandinöl abrialis | 10 | |
| Isobornylacetat | 3 | |
| Lysmeral⁴ | 10 | |
| Calone 1951⁵ | 2,5 | B |
| Florhydral² | 1,5 | B |
| Florol⁹ | 12 | A |
| Tetrahydrolinalool | 75 | A |
| Geraniumöl | 5 | |
| Isodamascon¹ (1-(2,4,4-Trimethyl-2-cyclohexen-1-yl)-2-buten-1-on), 10% in DPG | 2 | H |
| Resedafol¹ 10% in DPG [2-(1-Propoxyethoxy)ethyl]benzol | 1 | H |
| Methyldihydrojasmonat (Hedione⁹ HC) | 180 | A |
| Jessemal³ (3-Butyl-5-methyl tetrahydropyran-4-yl-acetat) | 4 | |
| Benzylsalicylat | 10 | A |
| alpha-Isomethylionon | 8 | A |
| Anethol | 3 | M |
| Methylcedrylketon | 50 | |
| Iso E Super³ | 25 | A |
| Ambrocenide¹ ((4aR,5R,7aS,9R)-Octahydro-2,2,5,8,8,9a-hexamethyl-4H-4a,9-Methanoazuleno(5,6-d)-1,3-dioxol), 1% in DPG | 3 | H |
| Timberol¹ (1-(2,2,6-Trimethylcyclohexyl)hexan-3-ol) | 2 | |
| Patchouliöl | 3,5 | |
| Evernyl² (Methyl-2,4-dihydroxy-3,6-dimethylbenzoat) | 1,5 | |
| Labdanum absolue 20% in DPG | 2 | H |
| Amber Core⁶ | 10 | |
| Ambraketal² | 1,5 | |
| Hydroxyambran⁷ (2-Cyclododecylpropanol), 50% in DPG | 5 | H |
| Macrolide¹ | 35 | A |
| Globalide¹ | 20 | A |
| Isopropylmyristat | 210 | H |
| Summe: | 1.000 | |

| | | |
|---|---|---|
| DPG = Dipropylenglycol ¹ Handelsname Symrise GmbH, Deutschland; ² Handelsname Givaudan AG, Schweiz; ³ Handelsname International Flavors & Fragrances Inc., USA; ⁴ Handelsname BASF AG, Deutschland; ⁵ Handelsname Danisco Seillans S.A., Frankreich; ⁶ Handelsname Kao Corp., Japan; ⁷ Handelsname Miltitz Aromatics, Deutschland. | | |

### Beispiel 2. Erfindungsgemäßer Akkord zur Erzielunq eines unmittelbar wahrnehmbaren Sauberkeits- und Reinlichkeitseffekts

Es werden vermischt:

| Komponente | Gew.-teile | Gruppe |
|---|---|---|
| L-Menthylmethylether | 200 | |
| L-Menthol | 200 | M |
| Methyldihydrojasmonat | 50 | A |
| Iso E Super³ | 100 | A |
| Dihydromyrcenol | 200 | A |
| 3-(4-Ethylphenyl)-2,2-dimethylpropanal (Florazon) | 10 | B |
| Dipropylenglycol | 240 | H |

### Beispiel 3. Herstellung eines Deodorant-Sprays auf alkoholischer Basis

In 49 Gew.-teilen 96-prozentigem Ethanol werden jeweils gelöst:
a) 1 Gew.-teil des in Beispiel 1 hergestellten Parfümöls
b) 0,8 Gew.-teile des in Beispiel 1 hergestellten Parfümöls und 0,2 Gew.-teile des in Beispiel 2 hergestellten Akkords.

Aus jeweils 1 Gew.-teil der resultierenden Lösung und 1 Gew.-teil eines Treibgases (Propan/Butan; Druck 2,5 bis 2,7 bar) wird ein Aerosolspray hergestellt, das als Deodorantspray an zahlreichen Probanden zur Bekämpfung von existierendem Achselschweißgeruch eingesetzt wurde.

Die Auswertung durch ein geschultes Sensorik-Panel ergab, dass das Deodorans, das die Mischung b) enthielt, eine schneller wahrnehmbare schweißgeruchüberdeckende und damit einen schneller wahrnehmbares Sauber-und Reinlichkeitsgefühl vermittelte als das Deodorans, das die Mischung a) enthielt.

Auch die Probanden, die die Wirkung der Parfümöle am eigenen Körper bewerten sollten, bemerkten ein schneller einsetzendes und verstärktes Sauber-und Reinlichkeitsgefühl bei Verwendung des Deodorans, das die Mischung b) enthielt.

### Bewertung der Geruchsverstärkung (Enhancer- bzw. Booster-Wirkung)

In einem weiteren Versuch wurden die beiden wie oben beschrieben hergestellten Aerosolsprays zahlreichen Probanden jeweils separat auf beide Unterarme gesprüht und beide Unterarme separat hinsichtlich der Geruchsstärke auf einer Skala von 1 (sehr schwach) bis 9 (sehr stark) bewertet und der jeweilige Mittelwert aller Bewertungen ermittelt.

Die Auswertung ergab, dass das Aerosolspray, das die Mischung b) enthielt, wesentlich stärker wahrgenommen (auf obiger Skala: 7) wurde als das Aerosolspray, das die Mischung a) enthielt (auf obiger Skala: 5).

### 4. Herstellung eines Antiperspirant-Sprays

Zu einem Gemisch aus geeigneten Trägerstoffen bestehend aus 12,5 Gew.-teilen Miglyol 840 Gel B (Mischung aus Propylenglycoldicaprylat/-dicaprat, Steralkonium Hectorit und Propylencarbonat; Hersteller: Sasol), und 46,5 Gew.-teilen Siliconölen (Cyclomethicon, Dimethiconol; Hersteller: Dow Corning) werden 40 Gew.-teile Aluminiumchlorhydrat hinzugefügt. Wenn die Mischung homogen geworden ist, werden hinzugefügt:
a) 1 Gew.-teil des in Beispiel 1 hergestellten Parfümöls
b) 0,8 Gew.-teile des in Beispiel 1 hergestellten Parfümöls und 0,2 Gew.-teile des in Beispiel 2 hergestellten Akkords.

Aus jeweils 1 Gew.-teil der resultierenden Lösung und 3 Gew.-teilen eines Treibgases (Propan/Butan; Druck 2,5 bis 2,7 bar) wird ein Aerosolspray hergestellt, das als Antiperspirantspray an zahlreichen Probanden zur Bekämpfung von existierendem Achselschweißgeruch eingesetzt wurde.

Die Bewertung durch ein geschultes Sensorik-Panel zeigte, dass das Antiperspirans, das die Mischung b) enthielt, eine schneller wahrnehmbare Überdeckung des Schweißgeruchs bot als das entsprechende Produkt, das nur die Mischung a) enthielt. Entsprechend wurde das Antiperspirans mit der Mischung b) als wesentlich geeigneter im Hinblick auf die Erzeugung eines sofort einsetzenden Sauber- und Reinlichkeitsgefühls bewertet.

Auch die Probanden selbst, an denen die Antiperspirantien getestet wurden, fühlten sich bei Verwendung des Produktes, das die die Mischung b) enthielt, signifikant reinlicher und sauberer.

### Beispiel 5. Akkord zur Erzielung eines unmittelbar wahrnehmbaren Sauberkeits-und Reinlichkeitseffekts

| Komponente: | Gew.-teile | Gruppe |
|---|---|---|
| L-Menthylmethylether | 200 | |
| Florazon¹ | 10 | B |
| Frescomenthe² | 30 | B |
| Pfefferminzöl | 10 | M |
| L-Menthol | 200 | M |
| L-Carvon | 10 | M |
| Calone 1951⁵ | 2 | B |
| Hedione⁹ | 50 | A |
| Anethol | 5 | M |
| Iso E Super³ | 100 | A |
| Ambrinol S¹ | 3 | B |
| Isomenthylacetat | 50 | |
| Dipropylenglycol (DPG) | 330 | H |
| Summe: | 1.000 | |

### Beispiel 6. Akkord zur Erzielung eines unmittelbar wahrnehmbaren Sauberkeits-und Reinlichkeitseffekts

| Komponente | Gew.-teile | Gruppe |
|---|---|---|
| I-Menthylmethylether | 100,00 | |
| Hexenylacetat trans-3 | 0,25 | B |
| Hexenylacetat cis-3 | 0,25 | B |
| Vertocitral¹ | 1,00 | B |
| Stemone² | 0,50 | B |
| Styrolylacetat | 5,00 | A |
| cis-3,3,5-Trimethylcyclohexylacetat | 300,00 | B |
| Melonal² | 0,50 | B |
| Dihydromyrcenol | 200,00 | A |
| Orangenöl brasilianisch | 35,00 | B |
| Grapefruitöl | 15,00 | B |
| Claritone¹ | 1,50 | B |
| Oxane⁹ 10% in DPG | 2,00 | B, H |
| Eucalyptusöl globulus | 8,00 | M |
| Pfefferminzöl arvensis | 8,00 | M |
| Borneol | 1,00 | B |
| Helional³ | 10,00 | B |
| Florhydral² | 1,00 | B |
| Tetrahydrolinalool | 80,00 | A |
| Hedione⁹ | 30,00 | A |
| 2,6-Nonadienol 10% in IPM | 1,00 | B, H |
| Summe: | 800,00 | |

| | | |
|---|---|---|
| DPG = Dipropylenglycol; IPM: Isopropylmyristat | | |

### Beispiel 7. Deo-Stifte

| Komponente | A | B | C | D |
|---|---|---|---|---|
| | Gew.-% | Gew.-% | Gew.-% | Gew.-% |
| Natriumstearat | 7,00 | 8,00 | 7,00 | 7,00 |
| Natriumpalmitat | 1,00 | - | - | 0,50 |
| 1,2-Propylenglycol | 42,00 | 45,00 | 44,00 | 48,00 |
| 1,2-Butylen glycol | 3,00 | - | 2,00 | - |
| 4-Methyl-4-phenyl-2-pentanol | 0,50 | 0,25 | 0,30 | - |
| 2-Butyloctansäure | - | 0,50 | 0,10 | 0,20 |
| 2-Hexyldecansäure | 0,10 | - | - | 0,20 |
| Polyethylenglycol(25)cetear ylether | 3,00 | 3,00 | - | - |
| Ethanol | 20,00 | 20,00 | - | - |
| Farnesol | - | 0,25 | 0,50 | - |
| Triclosan® (5-Chlor-2-(2,4-dichlorphenoxy)phenol) | 0,30 | - | - | 0,30 |
| Parabene (Mischung aus Methyl-, Ethyl-, Propyl-, Butyl-, Isobutylparaben) | 0,30 | - | 0,40 | - |
| 1,2-Hexandiol / 1,2-Octandiol (1:1) | - | 0,50 | - | 0,50 |
| 1,2-Pentandiol | 0,50 | - | 2,00 | - |
| Akkord nach Bsp. 5 | 1,50 | 0,35 | 1,20 | - |
| Akkord nach Bsp. 6 | - | 0,95 | - | 2,00 |
| Wasser | Ad 100 | Ad 100 | Ad 100 | Ad 100 |

### Beispiel 8. Transparente Deo-Stifte (Formulierunqen A, B) bzw. Deo-Creme-Stifte (Formulierunqen C, D)

| Komponente | A | B | C | D |
|---|---|---|---|---|
| | Gew.-% | Gew.-% | Gew.-% | Gew.-% |
| Aluminium Zirconium Tetrachlorohydrat - Glycin Komplex | 25,00 | 20,00 | 25,00 | 20,00 |
| Dimethicon (10 Cst) | - | - | 5,00 | 5,00 |
| Cyclopentasiloxan | - | 0,50 | 1,00 | 0,50 |
| Petrolatum | 5,00 | 4,70 | 5,00 | 5,00 |
| Ozokerit | 1,00 | 1,50 | - | - |
| Stearylalkohol | 12,00 | 12,00 | - | - |
| 2-Butyloctansäure | 0,50 | - | 0,50 | - |
| Wachs | - | - | 1,25 | 1,25 |
| PPG-14 Butylether | 9,00 | 9,00 | - | - |
| Gehärtetes Rapsöl | - | - | 5,00 | 5,00 |
| Siliciumdioxid | - | - | 1,00 | - |
| Farnesol | 0,25 | - | 0,25 | - |
| Paraffinöl | 0,50 | 0,50 | - | - |
| Hydriertes Rizinusöl (castor wax) | 3,50 | 3,50 | - | - |
| Talk | 4,00 | 4,00 | - | - |
| Behenylalkohol | 0,20 | 0,20 | - | - |
| d-Panthenyltriacetat | 1,00 | 1,00 | - | - |
| Konservierungsmittel | q.s. | q.s. | q.s. | q.s. |
| Akkord nach Bsp. 5 | 1,25 | - | 1,25 | - |
| Akkord nach Bsp. 6 | - | 0,25 | - | 1,00 |
| Parfümöl nach Bsp. 1 | - | 0,80 | - | 0,10 |
| Akkord nach Bsp. 2 | - | 0,20 | - | 0,25 |
| Wasser | Ad 100 | Ad 100 | Ad 100 | Ad 100 |

| | | | | |
|---|---|---|---|---|
| PPG: Polypropylenglycol | | | | |

### Beispiel 9. Antiperspirant-Roll-On

| Komponente | Gew.-% | Gew.-% |
|---|---|---|
| Caprylyl Trimethicon (SilCare TM Silicone 31 M 50) | 0,30 | 0,30 |
| Steareth-20 (GENAPOL TM HS 200) | 3,00 | 3,00 |
| Steareth-2 (GENAPOL TM HS 020) | 1,50 | 1,50 |
| Dicaprylyl Ether (Cetiol TM OE) | 2,00 | 2,00 |
| Coco Caprylat/Caprat (Cetiol TM LC) | 2,00 | 2,00 |
| Glycerin | 2,00 | 2,00 |
| Glyceryl Stearat (Cutina TM GMS) | 2,00 | 2,00 |
| Octyldodecanol (Eutanol TM G) | 1,00 | 1,00 |
| Stearyl alkohol | 2,50 | 2,50 |
| Aluminiumchlorohydrat gemäß Beispiel 1 der EP 1321431 | 10,00 | 10,00 |
| Avocado Extract Persea Gratissima | 0,30 | 0,20 |
| Akkord nach Bsp. 5 bzw. 6 | 0,50 | - |
| Parfümöl nach Bsp. 1 | - | 0,60 |
| Akkord nach Bsp. 2 | - | 0,15 |
| Wasser | Ad 100 | Ad 100 |

### Beispiel 10. Antiperspirant-Stick

| Komponente | Gew.-% | Gew.-% | Gew.-% |
|---|---|---|---|
| Phenyl Trimethicon (SilCare TM Silicone 15 M 50) | 13,50 | 13,50 | 13,50 |
| Cetearyl Alkohol | 19,30 | 19,30 | 19,30 |
| Cetiol CC (Dicaprylyl Carbonat) | 13,50 | 13,50 | 13,50 |
| Stearinsäure | 3,50 | 3,50 | 3,50 |
| PEG-40-Hydriertes Rizinusöl (Emulsogen TM HCO 040) | 4,10 | 4,10 | 4,10 |
| PEG-8 Distearate (Cithrol 4 DS) | 4,10 | 4,10 | 4,10 |
| Petrolatum | 6,90 | 6,90 | 6,90 |
| Aluminiumchlorohydrat | 13,80 | 13,80 | 13,80 |
| Aluminium Zirconium Trichlorohydrex Gly | 20,00 | 19,50 | 20,00 |
| 2,2-Dimethyl-3-phenylpropanol (Muguetalkohol¹) | - | 0,25 | - |
| Ethylhexylglycerin (Octoxyglycerin) | - | 0,30 | 0,20 |
| 2-Methyl-5-phenylpentan-1-ol (Rosaphen¹) | 0,30 | - | - |
| 4-Methyl-4-phenyl-2-pentanol (Vetikol¹) | - | 0,25 | 0,10 |
| Akkord nach Bsp. 5 bzw. 6 | 1,00 | 1,00 | - |
| Parfümöl nach Bsp. 1 | - | - | 0,80 |
| Akkord nach Bsp. 2 | - | - | 0,20 |

### Beispiel 11. Antiperspirant-Spray

| Komponente | Gew.-% | Gew.-% | Gew.-% |
|---|---|---|---|
| Glycerylstearat | 2,50 | 2,50 | 2,50 |
| Cetearyl Alkohol | 1,00 | 1,00 | 1,00 |
| Ceareth-20 | 2,50 | 2,50 | 2,50 |
| Ceareth-12 | 1,00 | 1,00 | 1,00 |
| Dicaprylyl Ether | 5,00 | 5,00 | 5,00 |
| Coco Caprylat/Caprat | 5,00 | 5,00 | 5,00 |
| Coffein | 0,25 | 0,25 | 0,25 |
| Phenoxyethanol | 0,25 | 0,25 | 0,25 |
| Parabene (Mischung aus Methyl-, Ethyl-, Propyl-, Butyl-, Isobutylparaben) | 0,25 | 0,25 | 0,25 |
| 1,2-Pentandiol | 2,00 | 1,00 | 1,20 |
| Aluminiumchlorohydrat | 8,00 | 8,00 | 8,00 |
| 4-Methyl-4-phenyl-2-pentanol (Vetikol¹) | 0,30 | 0,10 | 0,10 |
| Ethylhexylglycerin (Octoxyglycerin) | - | 0,30 | 0,50 |
| Akkord nach Bsp. 6 | 1,00 | - | 0,15 |
| Parfümöl nach Bsp. 1 | - | 0,75 | 0,50 |
| Akkord nach Bsp. 2 | - | 0,25 | 0,15 |
| Wasser | Ad 100 | Ad 100 | Ad 100 |

### Beispiel 12. Aerosolspray

| Komponente | Gew.-% | Gew.-% | Gew.-% |
|---|---|---|---|
| Octyldodecanol | 0,50 | - | 0,50 |
| Phenoxyethanol | - | - | 0,30 |
| 1,2-Pentandiol | 1,00 | 1,00 | 0,50 |
| 1,2-Hexandiol | 0,25 | 0,15 | - |
| 1,2-Octandiol | 0,25 | 0,25 | - |
| Farnesol | 0,25 | - | 0,25 |
| 2-Methyl-5-phenylpentan-1-ol (Rosaphen¹) | 0,10 | 0,15 | - |
| 4-Methyl-4-phenyl-2-pentanol (Vetikol¹) | 0,30 | - | 0,10 |
| Ethylhexylglycerin (Octoxyglycerin) | - | 0,30 | 0,50 |
| Akkord nach Bsp. 5 | 1,00 | - | 0,80 |
| Parfümöl nach Bsp. 1 | - | 0,55 | 0,10 |
| Akkord nach Bsp. 2 | - | 0,25 | 0,15 |
| Ethanol | Ad 100 | Ad 100 | Ad 100 |

Die nach Zusammenmischen der jeweils angegebenen Komponenten erhaltene flüssige Mischung wurde mit einem Propan-Butan-Gemisch (2:7= im Verhältnis 2 : 3 in einen Aerosolbehälter abgefüllt.

### Beispiel 13. Pump-Spray

| Komponente | Gew.-% | Gew.-% | Gew.-% |
|---|---|---|---|
| PEG-40-Hydriertes Rizinusöl | 2,00 | 2,00 | 2,00 |
| Glycerin | 1,00 | 1,00 | 1,00 |
| 1,2-Pentandiol | - | 1,00 | 2,00 |
| 1,2-Hexandiol | 0,25 | 0,15 | - |
| 1,2-Octandiol | 0,25 | 0,15 | - |
| Anisalkohol | 0,15 | 0,10 | 0,25 |
| 2-Methyl-5-phenylpentan-1-ol (Rosaphen¹) | - | 0,15 | - |
| 4-Methyl-4-phenyl-2-pentanol (Vetikol¹) | 0,30 | 0,15 | 0,10 |
| Ethylhexylglycerin (Octoxyglycerin) | - | - | 0,40 |
| Akkord nach Bsp. 5 | 0,80 | - | 0,80 |
| Parfümöl nach Bsp. 1 | - | 0,55 | 0,10 |
| Akkord nach Bsp. 2 | - | 0,25 | 0,15 |
| Wasser | Ad 100 | Ad 100 | Ad 100 |

### Beispiel 14. Roll-on-Gel

| Komponente | Gew.-% | Gew.-% | Gew.-% |
|---|---|---|---|
| PEG-40-Hydriertes Rizinusöl | 2,00 | 2,00 | 2,00 |
| 1,3-Butylenglycol | 2,00 | 2,00 | 2,00 |
| Hydroxyethylcellulose | 0,50 | 0,50 | 0,50 |
| 1,2-Pentandiol | - | 1,00 | 2,00 |
| 1,2-Hexandiol | 0,25 | 0,15 | - |
| 1,2-Octandiol | 0,25 | 0,15 | 0,25 |
| Phenoxyethanol | 0,15 | 0,10 | 0,25 |
| 2-Methyl-5-phenylpentan-1-ol (Rosaphen¹) | - | 0,15 | - |
| 4-Methyl-4-phenyl-2-pentanol (Vetikol¹) | 0,20 | 0,15 | 0,10 |
| Ethylhexylglycerin (Octoxyglycerin) | 0,50 | - | - |
| Akkord nach Bsp. 5 | 0,90 | - | 1,00 |
| Parfümöl nach Bsp. 1 | - | 0,55 | - |
| Akkord nach Bsp. 2 | - | 0,25 | 0,15 |
| Wasser | Ad 100 | Ad 100 | Ad 100 |

### Beispiel 15. Roll-on-Emulsion

| Komponente | Gew.-% | Gew.-% | Gew.-% |
|---|---|---|---|
| Triceteareth-4 Phosphat | 0,30 | 0,30 | 0,30 |
| Octyldodecanol | 2,00 | 2,00 | 2,00 |
| C₁₂₋₁₅-Alkylbenzoat | 2,00 | 2,00 | 2,00 |
| C₁₀₋₃₀-Alkylacrylat | 0,15 | 0,15 | 0,15 |
| 1,2-Hexandiol /1,2-0ctandiol (1:1) | - | 0,50 | 0,25 |
| Phenoxyethanol | 0,25 | 0,25 | - |
| Parabene | 0,25 | - | 0,20 |
| 2-Methyl-5-phenylpentan-1-ol (Rosaphen¹) | - | 0,15 | - |
| 4-Methyl-4-phenyl-2-pentanol (Vetikol¹) | 0,20 | 0,15 | 0,10 |
| Ethylhexylglycerin (Octoxyglycerin) | 0,50 | - | - |
| Akkord nach Bsp. 5 | 0,90 | - | 1,00 |
| Parfümöl nach Bsp. 1 | - | 0,55 | - |
| Akkord nach Bsp. 2 | - | 0,25 | 0,15 |
| Wasser | Ad 100 | Ad 100 | Ad 100 |

### Beispiel 16. Wachsstift

| Komponente | Gew.-% | Gew.-% | Gew.-% |
|---|---|---|---|
| Hydriertes Rizinusöl | 5,00 | 5,00 | 5,00 |
| Bienenwachse | 6,00 | 6,00 | 6,00 |
| C₁₂₋₁₅-Alkylbenzoat | 17,00 | 17,00 | 17,00 |
| Ceresin | 30,00 | 30,00 | 30,00 |
| 1,2-Hexandiol / 1,2-Octandiol (1:1) | - | 0,50 | 0,25 |
| Phenoxyethanol | 0,50 | - | - |
| Parabene | - | - | 0,25 |
| 4-Methyl-4-phenyl-2-pentanol (Vetikol¹) | 0,20 | 0,35 | 0,10 |
| 2,2-Dimethyl-3-phenylpropanol (Muguetalkohol¹) | 0,30 | - | 0,25 |
| Akkord nach Bsp. 5, verkapselt in beta-Cyclodextrin, Gehalt: 25 Gew.-% | 1,90 | - | 0,80 |
| Parfümöl nach Bsp. 1 | - | 0,55 | - |
| Akkord nach Bsp. 2 | - | 0,25 | 0,15 |
| Octyldodecanol | Ad 100 | Ad 100 | Ad 100 |

### Beispiel 17. Deo Zerstäuber

| Komponente | Gew.-% | Gew.-% | Gew.-% |
|---|---|---|---|
| PEG-40-Hydriertes Rizinusöl | 3,00 | 3,00 | 3,00 |
| Ethylhexylglycerin (Octoxyglycerin) | 0,80 | 0,80 | 1,00 |
| Ethanol | 40,00 | 40,00 | 40,00 |
| Citrat-Puffer | 0,50 | 0,50 | 0,50 |
| 1,2-Hexandiol / 1,2-Octandiol (1:1) | - | 0,25 | 0,25 |
| Phenoxyethanol | 0,25 | 0,25 | - |
| Triclosan® (5-Chlor-2-(2,4-dichlorphenoxy)phenol) | 0,25 | - | 0,20 |
| 2-Benzylheptan-1-ol (Jasmol¹) | | 0,25 | 0,15 |
| 4-Methyl-4-phenyl-2-pentanol (Vetikol¹) | 0,80 | 0,45 | 0,50 |
| Akkord nach Bsp. 5 | 0,80 | - | - |
| Akkord nach Bsp. 6 | - | - | 0,75 |
| Parfümöl nach Bsp. 1 | - | 0,65 | - |
| Akkord nach Bsp. 2 | - | 0,15 | |
| Wasser | Ad 100 | Ad 100 | Ad 100 |

### Beispiel 18. Roll-on

| Komponente | Gew.-% | Gew.-% | Gew.-% |
|---|---|---|---|
| Hydroxyethylcellulose | 0,50 | 0,50 | 0,50 |
| Ethylhexylglycerin (Octoxyglycerin) | 1,00 | 0,80 | 1,00 |
| Ethanol | 45,00 | 45,00 | 40,00 |
| Aluminium Chlorhydrat | 15,00 | 15,00 | 20,00 |
| 1,2-Hexandiol /1,2-Octandiol (1:1) | - | 0,25 | 0,25 |
| Phenoxyethanol | 0,25 | 0,25 | - |
| Parabene | 0,25 | - | 0,20 |
| 3-Methyl-6-phenyl-2-hexanol | - | 0,15 | 0,15 |
| 4-Methyl-4-phenyl-2-pentanol (Vetikol¹) | 0,40 | 0,80 | 0,50 |
| Akkord nach Bsp. 5 | 1,00 | - | - |
| Akkord nach Bsp. 6 | - | - | 0,85 |
| Parfümöl nach Bsp. 1 | - | 0,65 | - |
| Akkord nach Bsp. 2 | - | 0,15 | 0,15 |
| Wasser | Ad 100 | Ad 100 | Ad 100 |

### Beispiel 19. Emulsions-Roll-on

| Komponente | Gew.-% | Gew.-% | Gew.-% |
|---|---|---|---|
| PEG-40 Stearat | 5,00 | 5,00 | 5,00 |
| Ethylhexylglycerin (Octoxyglycerin) | 1,00 | 0,80 | 1,00 |
| Cetylalkohol | 2,00 | 2,00 | 2,00 |
| Aluminium Chlorhydrat | 15,00 | 15,00 | 15,00 |
| Polysorbat 80 | 1,00 | 1,00 | 1,00 |
| Glycerin | 1,50 | 1,50 | 1,50 |
| Mg-aluminium silicat | 0,80 | 0,80 | 0,80 |
| 1,2-Pentandiol | - | 0,50 | 1,00 |
| 2-Benzylheptan-1-ol (Jasmol¹) | - | 0,25 | - |
| 4-Methyl-4-phenyl-2-pentanol (Vetikol¹) | 0,50 | 0,50 | - |
| Akkord nach Bsp. 5 | 1,00 | - | - |
| Akkord nach Bsp. 6 | - | - | 0,85 |
| Parfümöl nach Bsp. 1 | - | 0,65 | - |
| Akkord nach Bsp. 2 | - | 0,15 | 0,15 |
| Wasser | Ad 100 | Ad 100 | Ad 100 |

### Beispiel 20. Roll-on

| Komponente | Gew.-% | Gew.-% | Gew.-% |
|---|---|---|---|
| Steareth 2 | 2,00 | 2,00 | 2,00 |
| Steareth 21 | 5,00 | 5,00 | 5,00 |
| Ethylhexylglycerin (Octoxyglycerin) | 1,00 | 0,80 | 1,00 |
| Cetearylalkohol | 0,50 | 0,50 | 0,50 |
| 1,2-Pentandiol | - | 0,50 | 1,00 |
| 4-Methyl-4-phenyl-2-pentanol (Vetikol¹) | 0,50 | 0,50 | - |
| Akkord nach Bsp. 5 | 1,00 | - | - |
| Akkord nach Bsp. 6 | - | - | 0,85 |
| Parfümöl nach Bsp. 1 | - | 0,65 | - |
| Akkord nach Bsp. 2 | - | 0,15 | 0,15 |
| Wasser | Ad 100 | Ad 100 | Ad 100 |

### Beispiel 21. Antiperspirant-Aerosol

| Komponente | Gew.-% | Gew.-% | Gew.-% |
|---|---|---|---|
| Silicone | 13,00 | 13,00 | 13,00 |
| Ethylhexylglycerin (Octoxyglycerin) | 0,80 | 0,80 | 1,00 |
| Ethanol | 0,80 | 0,80 | 0,80 |
| Quaternium-18 Hectorit | 0,80 | 0,80 | 0,80 |
| Aluminium Chlorhydrat | 10,00 | 10,00 | 10,00 |
| 1,2-Hexandiol /1,2-Octandiol (1:1) | 0,50 | 0,25 | 0,40 |
| Akkord nach Bsp. 5 | 1,00 | - | - |
| Akkord nach Bsp. 6 | - | - | 1,00 |
| Parfümöl nach Bsp. 1 | - | 0,80 | - |
| Akkord nach Bsp. 2 | - | 0,20 | - |
| Wasser | Ad 100 | Ad 100 | Ad 100 |

Quaternium-18 Hectorit (INCI-Bezeichung; CAS#: 71011-27-3):
quaternäre Ammoniumverbindungen, Bis(hydrierte Talg-alkyl)dimethyl-, Chloride, Verbindungen mit Hectorit

### Beispiel 22. Suspensions-Roll-on

| Komponente | Gew.-% | Gew.-% | Gew.-% |
|---|---|---|---|
| Silicone | 63,60 | 63,80 | 63,50 |
| Ethylhexylglycerin (Octoxyglycerin) | 1,00 | 1,00 | 1,00 |
| Quaternium-18 Hectorit | 13,00 | 13,20 | 13,70 |
| Aluminium Chlorhydrat | 21,00 | 20,00 | 20,00 |
| 4-Methyl-4-phenyl-2-pentanol (Vetikol¹) | - | 0,80 | 0,80 |
| Akkord nach Bsp. 5 | 1,40 | - | - |
| Akkord nach Bsp. 6 | - | - | 1,00 |
| Parfümöl nach Bsp. 1 | - | 0,90 | - |
| Akkord nach Bsp. 2 | - | 0,30 | - |

### Beispiel 23. Suspensionsstick

| Komponente | Gew.-% | Gew.-% | Gew.-% |
|---|---|---|---|
| Stearylalkohol | 20,00 | 20,00 | 20,00 |
| Cyclomethicone | 52,50 | 52,50 | 52,40 |
| PPG-14 Butylether | 2,00 | 2,00 | 2,00 |
| Hydriertes Rizinusöl | 1,00 | 1,00 | 1,00 |
| Talk | 2,00 | 2,00 | 2,00 |
| Aluminium Chlorhydrat | 20,00 | 20,00 | 20,00 |
| Triclosan® (5-Chlor-2-(2,4-dichlorphenoxy)phenol) | 0,30 | - | 0,30 |
| Ethylhexylglycerin (Octoxyglycerin) | 0,50 | 0,80 | 0,50 |
| 4-Methyl-4-phenyl-2-pentanol (Vetikol¹) | 0,50 | 0,40 | 0,55 |
| 2,2-Dimethyl-3-phenylpropanol (Muguetalkohol¹) | 0,30 | - | 0,15 |
| Anisalkohol | - | - | 0,15 |
| 2-Methyl-5-phenylpentan-1-ol (Rosaphen¹) | 0,10 | 0,20 | - |
| Akkord nach Bsp. 5 | 0,80 | - | 0,80 |
| Parfümöl nach Bsp. 1 | - | 0,85 | - |
| Akkord nach Bsp. 2 | - | 0,25 | 0,15 |

### Beispiel 24: Air-freshener in Gelform

5 g Accurel (poröses Homo-Polypropylen Pulver mit 75% Hohlraumanteil, ein Produkt der Akzo Nobel Faser AG, Obernburg, Deutschland) werden mit 4 g Akkord aus Beispiel 2 und 11 g Parfümöl nach Beispiel 1 durch Mischen der drei Komponenten unter Vakuum beladen. Das resultierende Pulver wird anschließend bei Normaldruck mit 4,5 g Wasser verrührt (Mix 1). In einem anderen separaten Gefäß werden 2,5 g Carragheen, 0,3 g Chloracetamid und 0,5 g Calciumchlorid-Dihydrat in 62 g Wasser unter Erwärmen bis auf etwa 75 °C gelöst. In diese Lösung wird unter Rühren Mix 1 eingebracht und homogenisiert.

Die resultierende, noch warme Mischung wird in die gewünschte Form (Kugeln, Halbkugeln, Kissen, Zylinder, Quader, Würfel, Muscheln oder ähnliche) gegossen. Nach dem Abkühlen auf etwa 20°C werden Raumerfrischer in Gelform erhalten, deren Beladung an Akkord und Parfümöl bei etwa 20 Gew. -% liegt.

## Patentansprüche

1. Verwendung
(i) eines Ethers der Formel (I) oder
(ii) einer Mischung von zwei oder mehr unterschiedlichen Ethern der Formel (I) 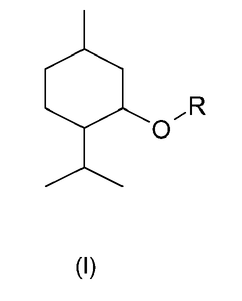
wobei R jeweils ein C₁ - C₄ - Alkyl darstellt,
zum Erzeugen eines Sauberkeits- und/oder Reinlichkeitsgefühls.

2. Verwendung nach Anspruch 1, wobei der, zumindest ein oder jeder Ether der Formel (I) jeweils ein Ether des d-Menthols, des I-Menthols oder ein Gemisch der Ether des racemischen Menthols ist.

3. Verwendung nach Anspruch 1 oder 2, wobei der, zumindest ein oder jeder Ether der Formel (I) jeweils ausgewählt ist aus der Gruppe bestehend aus I-Menthylmethylether, d-Menthylmethylether, dl-Menthylmethylether, I-Menthylethylether, d-Menthylethylether, dl-Menthylethylether, Menthyl-n-propylether, Menthyisopropylether und Menthylisobutylether.

4. Verwendung nach einem der vorangehenden Ansprüche, wobei das Sauberkeits- und/oder Reinlichkeitsgefühl erzeugt wird durch Wechselwirkung des, eines oder aller Ether der Formel (I), jeweils wie in einem der Ansprüche 1 bis 3 definiert,
(i) mit einem Körpergeruch, wobei die dem Körpergeruch eigene olfaktorische Wirkung abgeschwächt wird und/oder
(ii) mit einem Riechstoff, wobei die dem Riechstoff eigene olfaktorischen Wirkung verstärkt wird.

5. Verwendung nach Anspruch 4, wobei der Körpergeruch menschlicher Schweißgeruch, insbesondere Achselschweißgeruch, ist.

6. Gemisch, umfassend
(i) einen Ether der Formel (I), wie in einem der Ansprüche 1 bis 3 definiert, oder
(ii) eine Mischung von zwei oder mehr unterschiedlichen Ethern der Formel (I), jeweils wie in einem der Ansprüche 1 bis 3 definiert, und
(iii) wenigstens einen Riechstoff,
wobei der Anteil an dem oder den Ethern der Formel (I) so eingestellt ist, dass die dem Riechstoff eigene olfaktorische Wirkung verstärkt ist.

7. Gemisch nach Anspruch 6 oder Gemisch, umfassend
(i) einen Ether der Formel (I), wie in einem der Ansprüche 1 bis 3 definiert, oder
(ii) eine Mischung von zwei oder mehr unterschiedlichen Ethern der Formel (I), jeweils wie in einem der Ansprüche 1 bis 3 definiert, und
(iii) wenigstens einen Riechstoff,
wobei wenigstens ein Riechstoff ausgewählt ist aus der Gruppe (A), bestehend aus:
(A): alpha-Hexylzimtaldehyd, 2-Phenoxyethylisobutyrat (Phenirat), Dihydromyrcenol (2,6-Dimethyl-7-octen-2-ol), Methyldihydrojasmonat; vorzugsweise mit einem Gehalt an cis-Isomerem > 60 Gew.-% (Hedione, Hedione HC), 4,6,6,7,8,8-Hexamethyl-1,3,4,6,7,8-hexahydrocyclopenta[g]benzopyran (Galaxolid), Tetrahydrolinalool (3,7-Dimethyloctan-3-ol), Ethyllinalool, Benzylsalicylat, 2-Methyl-3-(4-tert-butylphenyl)propanal (Lilial), Zimtalkohol, 4,7-Methano-3a,4,5,6,7,7a-hexahydro-5-indenylacetat und/oder 4,7-Methano-3a,4,5,6,7,7a-hexahydro-6-indenylacetat (Herbaflorat), Citronellol, Vanillin, Linalylacetat, Styrolylacetat (1-Phenylethylacetat), Octahydro-2,3,8,8-tetramethyl-2-acetonaphthon und/oder 2-Acetyl-1,2,3,4,6,7,8-octahydro-2,3,8,8-tetramethylnaphtalin (Iso E Super), Hexylsalicylat, 4-tert.-Butylcyclohexylacetat (Oryclon), 2-tert.-Butylcyclohexylacetat (Agrumex HC), alpha-Ionon (4-(2,2,6-Trimethyl-2-cyclohexen-1-yl)-3-buten-2-on), n-alpha-Methylionon, alpha-Isomethylionon, Coumarin, Terpinylacetat, 4-(4-Hydroxy-4-methylpentyl)-3-cyclohexencarboxaldehyd (Lyral), alpha-Amylzimtaldehyd, Ethylenbrassylat, (E)-und/oder (Z)-3-Methylcyclopentadec-5-enon (Muscenon), 15-Pentadec-11-enolid und/oder 15-Pentadec-12-enolid (Globalide), 15-Cyclopentadecanolid (Macrolide), 1-(5,6,7,8-Tetrahydro-3,5,5,6,8,8-hexamethyl-2-naphthalenyl)ethanon (Tonalid), 2-Isobutyl-4-methyltetrahydro-2H-pyran-4-ol (Florol) und 2-Ethyl-4- (2,2,3-trimethyl-3-cyclopenten-1-yl)-2-buten-1-ol (Sandolen)
und/oder wenigstens ein Riechstoff ausgewählt ist aus der Gruppe (B), bestehend aus:
(B): cis-3-Hexenylacetat, trans-3-Hexenylacetat, trans-2,cis-6-Nonadienol, 2,4-dimethyl-3-cyclohexencarboxaldehyd (Vertocitral), 2,4,4,7-Tetramethyl-oct-6-en-3-on (Claritone), 5-Methyl-3-heptanonoxim (Stemone), 2,6-Dimethyl-5-hepten-1-al (Melonal), Borneol, 3-(3-Isopropylphenyl)butanal (Florhydral), 2-Methyl-3-(3,4-methylendioxyphenyl)propanal (Helional), 3-(4-Ethylphenyl)-2,2-dimethylpropanal (Florazon), 7-Methyl-2*H*-1,5-benzodioxepin-3(4*H*)-on (Calone 1951), 2-sec-Butylcyclohexanon (Frescomenthe), Orangenöl, Grapefruitöl, 3,3,5-Trimethylcyclohexylacetat (vorzugsweise mit einem Gehalt an cis-Isomeren größer oder gleich 70 Gew.-%), 2,5,5-Trimethyl-1,2,3,4,4a,5,6,7-octahydronaphthalin-2-ol (Ambrinol S), 2-Methyl-4-propyl-1,3-oxathiane (Oxane).

8. Gemisch nach Anspruch 7, wobei die gewichtsbezogene Gesamtmenge der Ether der Formel (I) oder des Ethers der Formel (I), jeweils wie in einem der Ansprüche 1 bis 3 definiert,
(i) zur gewichtsbezogenen Gesamtmenge der Riechstoffe der Gruppe (A) im Bereich von 15 : 1 bis 1: 50 liegt, und/oder
(ii) zur gewichtsbezogenen Gesamtmenge der Riechstoffe der Gruppe (B) im Bereich von 50 : 1 bis 1: 10 liegt.

9. Gemisch, umfassend
(i) einen Ether der Formel (I), wie in einem der Ansprüche 1 bis 3 definiert, oder
(ii) eine Mischung von zwei oder mehr unterschiedlichen Ethern der Formel (I), jeweils wie in einem der Ansprüche 1 bis 3 definiert, und
(iii) wenigstens ein Ausbringungshilfsmittel, ausgewählt aus der Gruppe (H), bestehend aus:
(H): Dipropylenglycol (DPG), Diethylphthalat (DEP), Triethylcitrat (TEC), Isopropylmyristat (IPM), und/oder Benzylbenzoat (BB).

10. Gemisch nach Anspruch 9, wobei das Gemisch gleichzeitig ein Gemisch nach einem der Ansprüche 6 bis 8 ist.

11. Gemisch nach Anspruch 9 oder 10, wobei die Gesamtmenge der Ether der Formel (I) oder des Ethers der Formel (I), jeweils wie in einem der Ansprüche 1 bis 3 definiert, zur Gesamtmenge Ausbringungshilfsmittel der Gruppe (H) im Bereich von 3 : 1 bis 1 : 100 liegt.

12. Gemisch nach einem der Ansprüche 6 bis 11, umfassend wenigstens eine Komponente, ausgewählt aus der Gruppe (M), bestehend aus:
(M): Eucalyptus-globulus-Öl, Pfefferminzöl, Krauseminzöl, Menthylacetat, Salbeiöl, I-Carvon, Menthol (vorzugsweise I-Menthol), Anethol, alpha-Terpineol, Geraniol, Linalool, 2-Phenylethylalkohol.

13. Parfümöl, umfassend ein Gemisch nach einem der Ansprüche 6 bis 12.

14. Parfümöl nach Anspruch 13, wobei die Gesamtmenge der Ether der Formel (I) oder des Ethers der Formel (I), jeweils wie in einem der Ansprüche 1 bis 3 definiert, bezogen auf die Gesamtmasse des Parfümöls im Bereich von 0,5 bis 30 Gew.-% liegt.

15. Kosmetische Zubereitung, umfassend ein Gemisch nach einem der Ansprüche 6 bis 12 oder ein Parfümöl nach Anspruch 13 oder 14.

16. Kosmetische Zubereitung nach Anspruch 15, wobei die kosmetische Zubereitung in einer Applikationsform vorliegt, ausgewählt aus der Gruppe bestehend aus Stiften, Cremes, Gelen, Lotionen, Schäumen, Roll-on-Präparaten, Pudersprays, Aerosol- oder Non-Aerosol-Sprays.

17. Kosmetische Zubereitung nach Anspruch 15 oder 16, wobei die Zubereitung ein Deodorant oder ein Antiaspirant ist.

18. Verwendung eines Gemisches nach einem der Ansprüche 6 bis 12 oder eines Parfümöls nach Anspruch 13 oder 14 oder einer Zubereitung nach einem der Ansprüche 15 bis 17 zum Erzeugen eines Sauberkeits- und/oder Reinlichkeitsgefühls.

19. Verfahren zum kosmetischen Erzeugen eines Sauberkeits- und/oder Reinlichkeitsgefühls, umfassend die Schritte:
a) Bereitstellen
(i) eines Ethers der Formel (I), wie in einem der Ansprüche 1 bis 3 definiert oder
(ii) einer Mischung von zwei oder mehr unterschiedlichen Ethern der Formel (I), jeweils wie in einem der Ansprüche 1 bis 3 definiert oder
(iii) eines Gemisches nach einem der Ansprüche 6 bis 12 oder
(iv) eines Parfümöls nach Anspruch 13 oder 14 oder
(v) einer Zubereitung nach einem der Ansprüche 15 bis 17, und
b) Applizieren des Ethers, der Mischung der Ether, des Gemisches, des Parfümöls oder der Zubereitung, so dass die olfaktorische Wirkung des Ethers, der Mischung der Ether, des Gemisches, des Parfümöls oder der Zubereitung so wahrnehmbar ist, dass ein Sauberkeits- und/oder Reinlichkeitsgefühls erzeugt wird.

20. Verfahren nach Anspruch 19, wobei das Applizieren auf die Haut erfolgt.

21. Verfahren nach einem der Ansprüche 19 oder 20, wobei das Applizieren durch Sprühen erfolgt.
